# EUROPEAN PATENT SPECIFICATION

(11) **EP 1 999 668 B1**
(45) Date of publication and mention of the grant of the patent: **30.07.2014**
(21) Application number: 07753209.1
(22) Date of filing: 15.03.2007
(51) Int. Cl.: C12N 15/10

(54) **METHODS OF SCREENING FOR AND MAPPING PHENOTYPIC AND GENOTYPIC VARIATIONS IN CELLS**
VERFAHREN FÜR DIE SUCHE NACH UND ABBILDUNG VON PHÄNOTYPISCHEN UND GENOTYPISCHEN VARIATIONEN IN ZELLEN
PROCEDES DE CRIBLAGE ET DE CARTOGRAPHIE DE VARIATIONS GENOTYPIQUES ET PHENOTYPIQUES DE CELLULES

(30) Priority: 15.03.2006 US 783102 P
(43) Date of publication of application: 10.12.2008
(73) Proprietor: Soper, Bryan, R., San Francisco CA 94107 (US)
(72) Inventor: Soper, Bryan, R., San Francisco CA 94107 (US)
(74) Representative: Roques, Sarah Elizabeth
(86) International application number: PCT/US2007/006563
(87) International publication number: WO 2007/106571

(56) References cited:
- WO-A2-2004/058050
- US-A1- 2004 018 624
- US-A1- 2004 018 624
- US-A1- 2004 117 865
- US-A1- 2005 227 233
- US-B1- 6 333 155
- US-B1- 6 928 368
- US-B1- 6 928 368
- BRANDA CATHERINE S ET AL: "Talking about a revolution: The impact of site-specific recombinases on genetic analyses in mice." DEVELOPMENTAL CELL JAN 2004 LNKD- PUBMED:14723844, vol. 6, no. 1, January 2004 (2004-01), pages 7-28, XP002579368 ISSN: 1534-5807
- STARK G R ET AL: "Forward genetics in mammalian cells: functional approaches to gene discovery" HUMAN MOLECULAR GENETICS, OXFORD UNIVERSITY PRESS, SURREY LNKD- DOI:10.1093/HMG/8.10.1925, vol. 8, no. 10, 1 January 1999 (1999-01-01), pages 1925-1938, XP002964457 ISSN: 0964-6906

## Description

### FIELD OF ENDEAVOR

Described herein are methods of determining in cultured cells the location of a genetic sequence correlated with genotypic or phenotypic variation, methods of identifying targets for drug discovery, methods of mutagenizing cells or cells in cell culture, and libraries of cells.

### BACKGROUND

Genetic mutations and chemical treatments are tools used to screen for altered phenotypes of cells and organisms. However, in diploid organisms or cells, a single mutation on one chromosome can be without phenotypic consequences where a wild type allele exists on the homologous chromosome. The phenotype of a recessive allele can be difficult to detect in cultured cells.

In order to study inherited congenital human diseases or recessive disease traits, scientists rely on studying inbred populations or linkage disequilibria. Each of these techniques counts on the phenotypic trait being fixed in a limited genetic pool. The rarity of isolated breeding populations limits the study of human genetics. A cultured cell-based screening system would enhance our ability to study the causes of disease, including but not limited to human disease.

Genetic model systems are used as a surrogate to understand human biology. Genotype of model organisms are manipulated and mutagenized to screen for phenotypic abnormalities. In diploid organisms such as *Drosophila melanogaster* and *Mus musculus,* chromosome deficiencies are exploited in genetic screens to identify new mutations in the portion of the genome that is hemizygous. Animals with phenotypic differences may be bred to map the causative genetic alteration to a discrete portion of the genome. However, screens conducted in model organisms or model animals are often costly and time consuming, and not necessarily representative of the actual organism for which the experimental organism is a model.

Embryonic stem (ES) cells may be used to make discrete genetic mutations on a single allele, and these mutations may be passed through the germ line to produce animals that can be bred to homozygosity. Where a mouse is bred to homozygosity for a recessive allele, the phenotype of the recessive allele may be observed. Some deletions as large as 3-4 centiMorgans (cM) have been reported in mouse ES cells and passed through the mouse germ cell line. Mice carrying these large deletions have been crossed to chemically mutagenized mice to aid in genetic screens in the animal offspring. Mapping of a genetic lesion of interest is typically done by breeding mutant organisms with genetically different organisms of the same species. The genetic crosses may assay genetic complementation, or may assay co-segregation of the genotypic or phenotypic variation, or carrier status, along with visible markers or physical markers. As above, mapping in organisms can be costly and time consuming, and a cultured cell-based system of mapping would enhance our ability to study the causes of diseases, including but not limited to human disease.

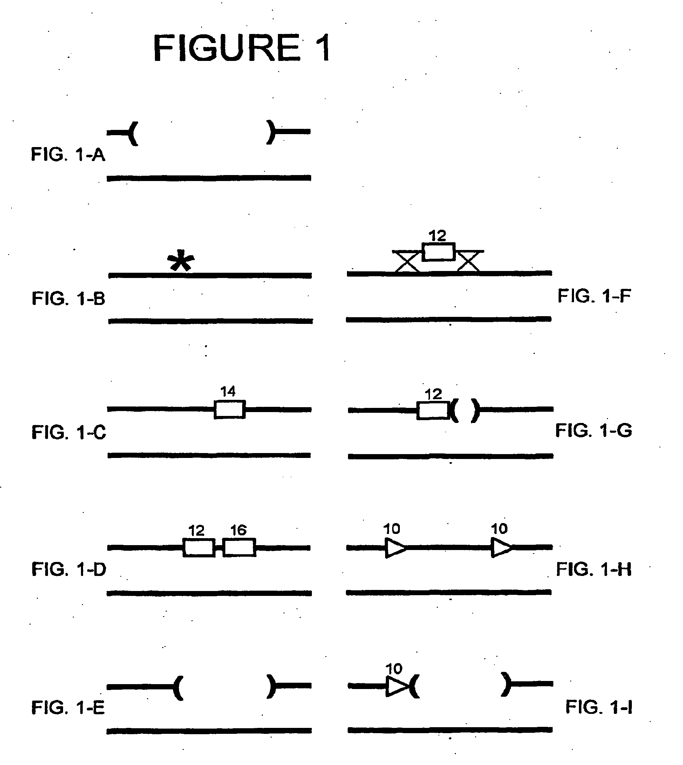

Treating cells with electromagnetic radiation, such as UV irradiation, X-rays, or gamma rays, causes several types of mutations, including deletions (FIG. 1-A). Treating cultured cells with chemical mutagens or inhibitors of DNA repair causes more discrete mutations, typically base substitutions, additions or deletions (FIG. 1-B). Treatment with electromagnetic radiation, chemical mutagens, or inhibitors of DNA repair typically results in heterozygous deletions throughout the genome. Thus, such methods when used alone are unlikely to uncover recessive mutations. Increasing the dosage or time of exposure to these mutagens could result in heteroallelic mutations, resulting in the loss of function of a gene. However, at mutagen levels causing the loss of two alleles of the same gene, lethality increases and the number of dual allelic mutations in the genome increases, complicating the screen and making it difficult to analyze the effect of any one allele. A large number of dual allelic mutations may also make it more difficult for investigators to identify the specific genetic lesion that causes the genotypic or phenotypic variation.

Insertional mutagenesis is another method of randomly or non randomly mutagenizing a genome. Insertional mutagens include, but are not limited to, transposons, retrotransposons, retroviruses, pro-viral constructs, transgenes, plasmids, repetitive sequence elements and gene traps. Insertional mutagens can be engineered to contain a positive selectable marker, or a visible marker. Since the point of insertion for many of these mutagens is somewhat random, they can disrupt a gene upon integration into the genome (FIG. 1-C). Insertional mutagens usually disrupt only a single allele, leaving a wild type allele intact. Again, such methods when used alone are unlikely to uncover recessive mutations. If the insertional mutagen contains a positive selectable marker, such as a drug selectable marker, then the cells can be selected at a higher drug concentration for the disruption of both alleles. However, this commonly results in additional concomitant rearrangements in the genome. This can result in other unknown mutations in the genome, which are responsible for a genotypic or phenotypic variation. This complicates the screen and makes it difficult to analyze the effect of any one genetic lesion. US 2004/018624 discloses a method of insertional mutagenesis for phenotype screening.

Some variations on mutagenizing the genome utilize the insertion of positive and negative drug selectable markers. Cells can be selected by, for example, treatment with a chemical or reagent, for the insertion of the positive selectable marker anywhere in the genome (FIG. 1-D). Then these cells can be treated with electromagnetic radiation, chemical mutagens, or inhibitors of DNA repair to cause deletions (FIG. 1-E). The deletions can be selected for by treating the cells with another drug to select against the presence of the negative selectable marker. The induced deletions are random and it requires mapping the breakpoints of each deletion. Once again this will typically create a deletion on one allele, while the wild type allele remains intact.

Instead of inducing random mutations throughout the genome, precisely defined mutations may be induced. Traditional gene targeting by homologous recombination (FIG. 1-F) often results in a small deletion, typically just one gene (FIG. 1-G). Similar to other methods this disrupts only a single allele, leaving a wild type allele intact. The inclusion of a positive drug selectable marker in the targeting cassette allows for selection of cells at higher drug concentration for the disruption of both alleles. This commonly results in additional concomitant rearrangements in the genome. This can result in other unknown mutations in the genome which result in a genotypic or phenotypic variation, thereby complicating the screen and making it difficult to analyze the effect of any one allele.

Another alternative is to include site-specific recombination sites in the targeting cassette, and to target the same chromosome twice (FIG. 1-H). The cells can then be treated with a site-specific recombinase, resulting in the deletion of intervening sequences (FIG. 1-I). Much larger deletions can be practically achieved in this manner than through a single targeting step. However, this only causes a deletion on one chromosome, while the other chromosome is wild type. The deletion of both alleles would require subsequent targeting of the other chromosome. Usually, discrete mutations made by homologous recombination are performed in ES cells. Adult animals are derived from the ES cells in order to breed mutations to homozygosity, or to utilize in genetic screens by crossing to other adult animals. Screens in organisms or animals are much more expensive and time consuming than screens in cell culture.

Another limitation of using cultured cells to screen for genotypic or phenotypic variation is the current inability to efficiently map genotypic or phenotypic variations to discrete genetic lesions. Mutant mammalian cells have previously been found in genetic screens for phenotypic variants, but the causative lesion in many such cells remains unknown.

Improved cell culture-based methods and tools for screening and identifying genetic sequences correlated with genotypic and phenotypic variations, such as recessive genotypic and phenotypic variations, are needed.

### SUMMARY

Described herein are methods of mutagenizing cells or cells in cell culture, methods of screening cells in cell culture, methods of mapping genetic lesions correlated with genotypic or phenotypic variation in cell culture, libraries of cells, and methods of using genetic sequences identified by the methods described herein.

Described herein are methods of screening for phenotypic variation in cells or organisms, including but not limited to eukaryotic cells or organisms, using genomic deletions in combination with other methods of generating change in the genetic sequence, gene product abundance, or gene product function, including but not limited to via mutagens, drugs, inhibitors of DNA repair, or methods that affect RNA or protein abundances or activities. Described herein are methods of mapping genetic sequences associated with a genotypic or phenotypic variation using cells identified in the screens described herein.

Described herein are methods of determining the location or identity of a genetic sequence correlated with a recessive genotypic or phenotypic variation in cultured cells, the genetic sequence located between two inserted site-specific recombination sites, comprising:
(a) obtaining cells comprising two inserted site-specific recombination sites on a chromosome, further comprising a mutation that is correlated with a genotypic or phenotypic variation, wherein the mutation is on a homologous chromosome and bounded by the location of the site-specific recombination sites on the homolog;
(b) inserting a third site-specific recombination site at a known location between the two site-specific recombination sites in a population of the cells of (a);
(c) exposing the cells of (a) to a site-specific recombinase to generate cells comprising at least one deletion;
(d) exposing the cells of (b) to a site-specific recombinase to generate cells comprising at least one deletion;
(e) screening in cultured cells or ex vivo the deletion-containing cells of (c) relative to the deletion-containing cells of (d) for the presence or absence of the phenotypic or genotypic variation;
(f) repeating steps (a) - (c), wherein the third site-specific recombination site is altered relative to that of the first iteration of steps (a) - (c);
(g) determining the location or identity of a genetic sequence that is correlated with the recessive genotypic or phenotypic variation in the cultured cells or ex vivo.

Described herein are methods wherein a third site-specific recombination site is altered relative to that of the first iteration of steps (a) - (c) to narrow the size of the deletion that is correlated with the genotypic or phenotypic variation in the cells, wherein the mutation is located at a site on the homologous chromosome corresponding to the deletion.

Described herein are methods of identifying a genotypic or phenotypic variation, comprising:
screening cells cultured or ex vivo for genotypic or phenotypic variation between:
   (a) greater than haploid cells with a recombinase-induced deletion between two inserted site-specific recombination sites in the chromosomal DNA of cultured cells, wherein the DNA of the cells comprises a further mutation in a chromosome homologous with at least a portion of the recombinase-induced deleted sequence, wherein the further mutation was introduced with a method other than recombinase-induced deletion; and .
   (b) cells selected from the group consisting of:
      (i) progenitor cells of the cells of (a) without the recombinase-induced deletion in the chromosomal DNA or the further mutation;
      (ii) progenitor cells of the cells of (a) having the recombinase-induced deletion in the chromosomal DNA but absent the further mutation,
      (iii) progenitor cells of the cells of (a) having the introduced further mutation but absent the recombinase-induced deletion in the chromosomal DNA, and
      (iv) cells other than progenitor cells of the cells of (a)
wherein the location or identity of the genotypic or phenotypic variation is localized to a region of a genetic sequence correlated with the genotypic or phenotypic variation using a method described herein.

In some variations the further mutation is via chemical mutagenesis. In some variations the further method of mutation of the cultured cells of (a) is performed either prior to, concurrently with, or subsequent to the recombinase-induced deletion. In some variations the cell is animal, plant, fungal, or protist. In some variations the cell comprises one or more reporter or selectable markers. In some variations the cells of (a) comprise one or more reporter or selectable markers internal to the two recombination sites: In some variations the cell comprises one or more reporter or selectable markers external to the two recombination sites.

In some variations the site-specific recombinase is Cre recombinase, FLP recombinase, or Int recombinase. In some variations the site-specific recombinase is Cre recombinase.

In some variations the cells prior to mutagenesis closely adhere to a diploid karyotype.

In some variations the further mutagenesis is by one or more of electromagnetic radiation, treatment with an inhibitor of DNA repair, or insertional mutagenesis.

In some variations the phenotypic variation is a member selected from the group consisting of an altered ability to grow colonies in semi-solid medium, altered resistance to chemotherapeutic agents, altered induction of neo-angiogenesis, and an altered ability to metastasize.

In some variations the cultured cells are mammalian cells. In some variations the cultured mammalian cells are primate, human, mouse or rat cells. In some variations the cultured mammalian cells are human ells.

In some variations the cells of (a) exhibit characteristics associated with a disease state.

In some variations the cells of (a) exhibit characteristics associated with a cancer. In some variations the cells of (a) exhibit characteristics associated with lung cancer. In some variations the cells of (a) are a member from the group consisting of DV90, HLF-a, H522, H460, H460SM, SCLC-R1, SCC-37, Ma-4, Ma-10, Ma-12, Ma-15, and 1a 162 cells.

In some variations the cells of (a) exhibit characteristics associated with a member from the group consisting of Parkinson's disease, Alzheimer's disease, and HIV.

In some variations the variation is a loss of a phenotype displayed by the cells of (a). In some variations the variation is a loss of a genotype displayed by the cells of (a).

Described herein are methods of determining the location or identity of a genetic sequence correlated with a recessive genotypic or phenotypic variation in cultured cells or ex vivo comprising:
(a) obtaining cells comprising an inserted first site-specific recombination site, further comprising a mutation that is correlated with a genotypic or phenotypic variation, wherein the mutation is on a homologous chromosome;
(b) introducing a second site-specific recombination site onto the chromosome comprising the first site-specific recombination site in a population of cells of (a) wherein the location of the mutation on the homologous chromosome is bounded by the location of the site-specific recombination sites on the homolog;
(c) exposing the cells of (b) to a site-specific recombinase to generate cells comprising at least one deletion;
(d) screening in cultured cells or ex vivo the cells of (c) for the phenotypic or genotypic variation relative to the cells of (a);
(e) repeating steps (b) - (d), wherein the location of the second site-specific recombination site is altered relative to that of the first iteration of steps (b) - (d); and
(f) determining the location or identity of the genetic sequence that is correlated with the genotypic or phenotypic variation in the cultured cells or *ex vivo.*

In some variations the location of the second site-specific recombination site is altered relative to that of the first iteration of steps (a) - (c) to narrow the size of the deletion that is correlated with the genotypic or phenotypic variation in the cells.

Described herein are methods of identifying a genetic sequence correlated with a recessive genotypic or phenotypic variation, comprising
screening in cell culture or *ex vivo* for genotypic or phenotypic variation between
(a) cells formerly comprising an introduced recombination site, the region of sequence comprising the introduced recombination site having been deleted, wherein the DNA of the cells further comprises a mutation on a homologous chromosome that was introduced with a second method, wherein the mutation is located at a site on the homologous chromosome corresponding to the deletion; and
(b) cells from the group consisting of:
   (i) progenitor cells of the cells of (a) without the non-recombinase-induced deletion in the chromosomal DNA and without the further mutation with the second method;
   (ii) progenitor cells of the cells of (a) having the non-recombinase-induced deletion of the recombination site in the chromosomal DNA but absent the further mutation,
   (iii) progenitor cells of the cells of (a) having the introduced further mutation but absent the non-recombinase-induced deletion of the recombination site in the chromosomal DNA, and
   (iv) cells other than progenitor cells of the cells of (a)
wherein the genetic sequence correlated with the recessive genotypic or phenotypic variation in the cells of (a) is mapped to a region of a genetic sequence correlated with the genotypic or phenotypic variation using a method described herein.

In some variations the second method of mutation is chemical mutagenesis. In some variations the non-recombinase-induced deletion was via electromagnetic radiation.

In some variations the cells are animal, plant, fungal, or protist. In some variations the cells comprise one or more reporter or selectable markers.

In some variations the cells of (a) comprise one or more introduced reporter or selectable markers 5' to the introduced recombination site. In some variations the cells of (a) comprise one or more introduced reporter or selectable markers 3' to the introduced recombination site.

In some variations the site-specific recombinase is Cre recombinase, FLP recombinase, or Int recombinase. In some variations the site-specific recombinase is Cre recombinase.

In some variations the cells prior to mutagenesis closely adhere to a diploid karyotype.

In some variations the further mutagenesis is by one or more of treatment with an inhibitor of DNA repair, or an insertional mutagen.

In some variations the phenotypic variation is a member selected from the group consisting of an altered ability to grow colonies in semi-solid medium, altered resistance to chemotherapeutic agents, altered induction of neo-angiogenesis, and an altered ability to metastasize.

Described herein is a population of cells, comprising:
(a) a population of greater than haploid cultured cells with three introduced site-specific recombination sites in the chromosomal DNA, and
(b) a further mutation on a homologous chromosome of the cells at a location bound by the sites homologous to two of the introduced recombination sites.

In some variations the cells have a deletion caused by site-specific recombination between two of the three introduced site-specific recombination sites in on a single chromosome. In some variations the site-specific recombination occurred after exposing the cells to a site-specific recombinase selected from the group consisting of Cre recombinase, FLP recombinase, or Int recombinase. In some variations the site-specific recombinase was Cre recombinase.

In some variations the cells closely adhere to a diploid karyotype.

In some variations the further mutation was by one or more members from the group consisting of chemical treatment, electromagnetic radiation, treatment with an inhibitor of DNA repair, and insertional mutagenesis.

In some variations the cells are animal, plant, fungal, or protist. In some variations the cells are animal cells. In some variations the animal cells are primate, human, mouse or rat cells. In some variations the animal cells are human cells.

In some variations the human cells comprise a cell line whose progenitor cell line exhibits characteristics associated with a disease state.

In some variations the progenitor cell line exhibits characteristics associated with cancer. In some variations the progenitor cell line is a lung cancer cell line. In some variations the lung cancer cell line is a member from the group consisting of DV90, HLF-a, H522, H460, H460SM, SCLC-R1, SCC-37, Ma-4, Ma-10, Ma-12, Ma-15, and 1a 162.

In some variations the progenitor cell lines exhibit characteristics associated with one or more of a member from the group consisting of Parkinson's disease, Alzheimer's disease, and HIV.

Described herein are cell libraries comprising cells described herein.

Described herein are compositions comprising a cultured or ex vivo cell comprising a deletion on each of a first and second homologous chromosome; wherein the deletion in the first chromosome overlaps a deletion in the second chromosome, and wherein at least one of the deletions was introduced. In some variations, the cell is a cultured cell. In some variations, the deletion on both the first and the second homologous chromosomes was introduced. In some variations the cultured or ex vivo cells are prepared by introducing a deletion on a homologous chromosome. In some variations, the deletion on both the first and the second homologous chromosomes was introduced. In some variations, at least one of the introduced deletions was randomly introduced. In some variations, at least one of the introduced deletions was a targeted deletion.

Described herein are methods of identifying a genotypic or phenotypic variation, comprising subjecting the cells described herein, cultured or ex vivo, to a condition, and analyzing the response of the cells in the composition.

Described herein are methods of identifying a genotypic or phenotypic variation, comprising screening cells cultured or ex vivo for genotypic or phenotypic variation between:
(a) greater than haploid cells with a first introduced site-specific recombination site in a first homologous chromosome and comprising a deletion in a chromosome region of a second homologous chromosome wherein the deletion in the second homologous chromosome overlaps the first introduced site-specific recombination site in the first homologous chromosome; and
(b) greater that haploid cells of (a) wherein the cells further comprise a deletion in a chromosome region between the first introduced site-specific recombination site and a second introduced site-specific recombination site in the first homologous chromosome, wherein the correlation of the presence or absence of the genotypic or phenotypic variation in the cells of (b) is correlated with the region of the deletion between the first introduced site- specific recombination site and the second introduced site-specific recombination site.

Described herein are methods of determining the location or identity of a genetic sequence correlated with a genotypic or phenotypic variation in cultured cells or ex vivo comprising:
(a) obtaining greater than haploid cells with a first introduced site-specific recombination site on a first homologous chromosome and comprising a deletion in a chromosome region of a second homologous chromosome, wherein the deletion in the second homologous chromosome overlaps the first introduced site-specific recombination site in the first homologous chromosome, wherein at least a portion of the deleted region is correlated with a genotypic or phenotypic variation;
(b) introducing a second site-specific recombination site onto the first homologous chromosome comprising the first site-specific recombination site in a population of cells of (a);
(c) exposing the cells of (a) to a site-specific recombinase to generate cells comprising at least one deletion on the first homologous chromosome comprising the first and second recombination sites;
(d) screening in cultured cells or ex vivo the cells of (c) for the phenotypic or genotypic variation relative to the cells of (a);
(e) repeating steps (a) - (d), wherein the region of genetic sequence containing the second site- specific recombination site is altered relative to that of the first iteration of steps (a) - (d); and
(f) determining the location or identity of the genetic sequence that is correlated with the genotypic or phenotypic variation in the cultured cells or ex vivo, wherein the presence or absence of the genotypic or phenotypic variation in the cells of (d) indicates the region of the deletion in the homologous chromosome that correlates with the genotypic or phenotypic variation.

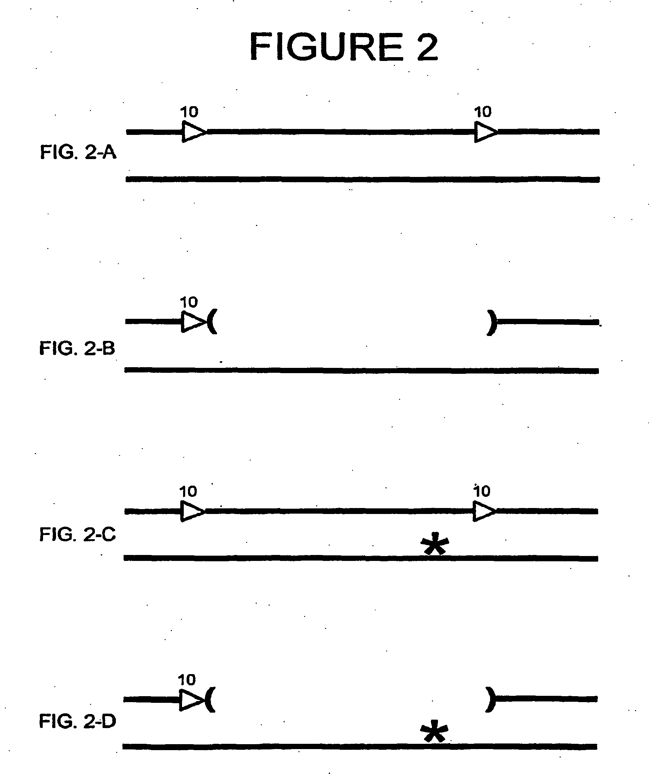

In some variations described herein, a first site-specific recombination site is introduced into cells, followed by a second site-specific recombination site. In some variations, the introduction of the site-specific recombination sites into the genome is by conventional homologous recombination methods. In other variations the method of introduction includes but is not limited to random integration, pro-viral insertion, gene trap, or transposition. In some variations, for a site-specific recombinase to cause a deletion between the site-specific recombination sites, the integration of both sites resides on the same chromosome in a certain orientation (FIG. 2-A). In such a variation, a site-specific recombinase may be introduced into a cell clone, and the presence of a deletion (FIG. 2-B) can be detected by standard means including, but not limited to, Southern blots, fluorescent *in situ* hybridization, multiplex fluorescent *in situ* hybridization, spectral karyotyping, comparative genomic hybridization, or molecular combing.

In some variations, cell clones without substantial deletions, but with correctly targeted site-specific recombination sites on the same chromosome (FIG. 2-A), are randomly mutagenized using chemical mutagens such as ethylmethane sulphonate (EMS) or ethylnitrosourea (ENU), or treated with inhibitors of DNA repair. In some variations, a plurality of individual clones resulting from such mutagenesis are segregated and grown (FIG. 2-C). In a further variation, clones are then treated with a site-specific recombinase to produce a deletion between the two site-specific recombination sites. Clones containing mutations and deletions may then be screened for any phenotypic variation (FIG. 2-D).

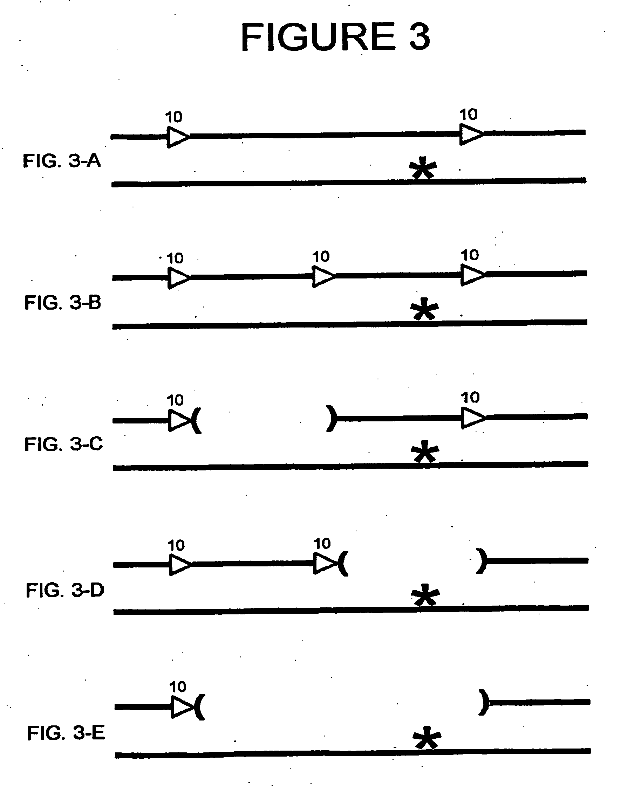

In some variations, phenotypic variant clones are selected and a third site-specific recombination site is introduced in the parental cell line (with the mutation, without the deletion) (FIG. 3-A), optionally between the previous two sites (FIG. 3-B). In this variation, cells are treated with a site-specific recombinase to create smaller deletions (FIGS. 3-C, 3-D). Cell clones are then re-screened for phenotypic variation. In a further variation, cell clones in which the mutation is not uncovered by the deletion on the homologous chromosome (FIG. 3-C) are not expected to display phenotypic variation. Cell clones in which the mutation is uncovered by the deletion on the homologous chromosome (FIG. 3-D) are expected to display phenotypic variation. In some variations, the deletion region is reiteratively narrowed by altering the location of the third site-specific recombination site (FIG. 3-B). In this manner, the deletion region can be narrowed until it is desirable or practical to sequence this interval of the chromosome from the phenotypic variant and the non-mutagenized parental cell as a control. Sequence differences are expected to correlate with genetic lesions responsible for phenotypic variation.

In some variations, cell clones with induced deletions (FIG. 2-B) are treated with mutagens or inhibitors of DNA repair. In a further variation, cell clones containing deletions and mutations (FIG. 2-D) are screened for genotypic or phenotypic variation. Individual cell clones (FIG. 2-D) may be screened for mutations in each gene within the interval bounded by site-specific recombination sites. In some variations the gene sequences are compared by sequencing, or by a hybridization method.

In some variations, deletions are caused by electromagnetic radiation.

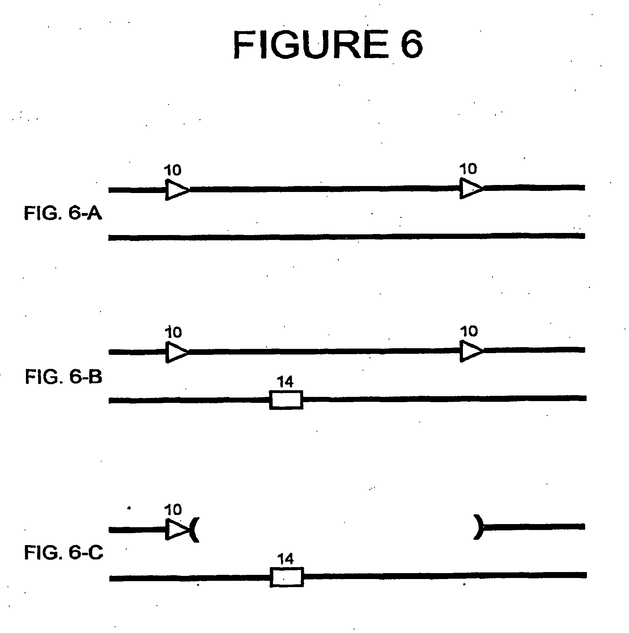

In some variations, mutations are produced by other insertional mutagens (FIG. 6), including but not limited to transposons, retrotransposons, retroviruses, pro-viral constructs, transgenes, plasmids, repetitive sequence elements and gene traps.

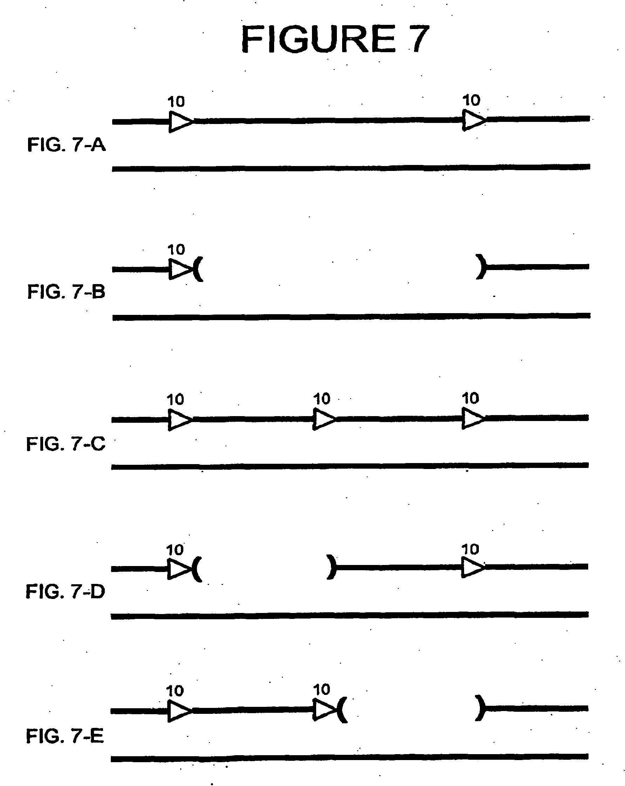

In some variations, cells containing deletions (FIG. 7-B) are treated directly with chemical compounds, including but not limited to compounds from chemical libraries, to identify phenotypic variant cells. The deletion regions can be narrowed to identify gene product targets (FIGS. 7-C, 7-D, 7-E).

In some variations, cells containing deletions (FIG. 7-B) are treated with methods to affect RNA or protein abundances or activities. In some variations, the abundance of one or more RNAs in reference cells (FIG. 7-A) is 100%, while cells containing deletions (FIG. 7-B) may have less than 100% abundance from genes deleted in the interval. In some variations, cells containing deletions have about 10%, about 20%, about 30%, about 40%, about 50%, about 60%, about 70%, about 80%, about 90% accumulation of the one or more RNAs as compared to reference cells. This improves the efficiency of screens altering RNA or protein abundances or activities.

In some variations, cells containing deletions (FIG. 7-B) are used to detect the mutagenicity of chemical compounds.

In some variations, cells containing deletions are used to detect the therapeutic effect of one or more therapeutic agents, including but not limited to chemical compounds, small molecules, nucleic acids, peptides, proteins, etc.

In some variations DNA containing site-specific recombination sites and/or positive and negative selectable markers is incorporated into cells. Deletions may be induced through, for example, the introduction of a site-specific recombinase or by treating with electromagnetic radiation. Cells may be screened for a changed response to drugs or other appropriate stimuli, for example via selection against a negative selectable marker. Cells containing deletions may be targeted on the corresponding homologous chromosome segment with site-specific recombination sites and/or positive and negative selectable markers. In some variations, deletions of the homologous chromosome segment are induced by introduction of a site-specific recombinase or by treating with electromagnetic radiation, and drugs or other appropriate stimuli are used to select against the negative selectable marker. In some variations, cell clones containing heteroallelic deletions may be screened for genotypic or phenotypic variation.

Described herein is a method of generating a library of cells with mutations in each gene. If the entire genome is targeted in this manner, a library of cells could be achieved with mutations in every gene. Theoretically, this library could comprise cells which collectively have a mutation in every gene, and the clones could be screened for any genotypic or phenotypic variation of interest.

### BRIEF DESCRIPTION OF THE DRAWINGS

FIG. 1 depicts some variations of methods of generating lesions in DNA.
FIG. 2 depicts some variations of methods for combining deletions in one chromosome with chemically induced mutations in a homologous chromosome.
FIG. 3 depicts some variations of methods for localization of the genetic lesion in one chromosome by using smaller deletions in a homologous chromosome.
FIG. 4 depicts some variations of a detailed view of the modifications targeted on one chromosome, to facilitate the induction of deletions on the chromosome.
FIG. 5 depicts some variations of additional modifications targeted to the chromosome in FIG. 6, to facilitate the induction of smaller deletions on the chromosome.
FIG. 6 depicts some variations of a method of using insertional mutagenesis on one chromosome in combination with a deletion on the homologous chromosome.
FIG. 7 depicts some variations of methods for an alternative way of using the deletion-containing cells in combination with drug treatment, in combination with non-limiting exemplary methods of affecting RNA or protein abundances or activities, to screen for phenotypic variation in cultured cells.
FIG. 8 depicts some variations of methods for an alternative way of creating deletions in cells using electromagnetic radiation along with using selectable markers.
FIG. 9 depicts some variations of methods for localization of the genetic lesion in one chromosome by using targeted deletions in a homologous chromosome.
FIG. 10 depicts some variations of methods for localization of the genetic lesion in one chromosome by using targeted deletions in the homologous chromosome.
FIG. 11 depicts some variations of methods for localization of the genetic lesion in one chromosome by using targeted deletions in a homologous chromosome.
FIG. 12 depicts some variations of methods for combining deletions in one chromosome with deletions in a homologous chromosome.
FIG. 13 depicts some variations of methods for an alternative way of creating deletions in one chromosome with deletions in a homologous chromosome, and for localization of the genetic lesion in one chromosome by using smaller deletions in a homologous chromosome.
FIG. 14 depicts some variations of methods for an alternative way of combining deletions in one chromosome with deletions in a homologous chromosome.
FIG. 15 depicts some variations of methods for an alternative way of localization of the genetic lesion in one chromosome by using smaller deletions in a homologous chromosome.

### DRAWINGS - FIGURE REFERENCE NUMERALS AND SYMBOLS

| | | | | | |
|---|---|---|---|---|---|
| 10 | site-specific recombination site | 12 | | positive selectable marker | |
| 14 | insertional mutagen | 16 | | negative selectable marker | |
| 18 | visible marker | * | mutation | ( ) | deletion |

### DETAILED DESCRIPTION

Provided herein are cells and methods of screening for and mapping genotypic or phenotypic variation of cells in cell culture. The methods described herein facilitate saturation mutagenesis of the genome in cultured cells, and mapping causative genetic lesions in cultured cells.

### Advantages of Cell Culture-Based Methods

The ability to use cultured cells for identifying and mapping genetic lesions, as opposed to whole organisms, offers powerful advantages over the use of whole organisms or animals. One advantage is a decreased amount of time required for obtaining answers, such as the identity of a target for therapy relating to a disease. Cultured cells are more quickly transformed or mutagenized than whole organisms or animals. Cultured cells are easily cloned, and the doubling time is measured in hours, as compared to months in the case of mice, or decades in the case of humans. Another advantage is the money it takes to obtain answers. The cost of culturing cells is a fraction of the cost of maintaining mice or other organisms traditionally used as genetic model systems. The cost of conducting screens on cells in culture is much lower than screening whole organisms for genotypic or phenotypic variation. Other advantages are convenience and flexibility. Cultured cells can be cryogenically frozen, thawed when desired, and available for experiments within days. Also, cell clones can be archived at each step of manipulation, so parental or grandparental clones can be re-thawed for further analysis. Further, if desired, transgenic organisms can be generated from the mutagenized cells to analyze the effect of the mutation in the organism. Exemplary methods to recover mutant cells are described in U.S. Pat. No. 4,818,679.

The combinations of mutagenesis methods described herein allow for identification of genotypic or phenotypic variation, including recessive genotypic or phenotypic variation, by screening cells in cell culture, and mapping the genetic lesion correlated with the genotypic or phenotypic variation in cultured cells.

Genetic sequences or lesions identified as involved in a particular disease or condition, including but not limited to cancer, may be used as targets in screens for therapeutic agents, including but not limited to one or more of small molecule therapies or gene therapies. Thus, among other benefits, the methods described herein offer the potential for faster, easier, and cheaper cures for human and veterinary diseases and conditions.

The ability to identify all of the genotypic variation involved in a process is "saturation mutagenesis." Model genetic organisms like fruit flies, which have a short generation time, and a low cost and space requirement for housing, offer the benefit of saturation mutagenesis. However, saturation mutagenesis is not a practical reality in other model organisms such as mice because of the generation time, litter size, and cost of housing. The methods described herein make saturation mutagenesis possible in a vastly expanded number of organisms, including but not limited to eukaryotic organisms, since the mutagenesis and phenotypic screening can be done in cell culture rather than in multicellular organisms, and the genetic lesion can be more easily identified.

Screening for genetic lesions is sometimes done in stem cells or cells that can be manipulated to allow passage through the germ line, which allows for the use of conventional genetic breeding to map the mutations. However, this is costly and time consuming. In some variations, cells mutagenized by the methods described herein, or parental cells thereof, are subjected to a reiterative deletion process in cultured cells to map discrete lesion in the genome. This can reduce the time and cost of mapping the lesions responsible for genotypic or phenotypic variation. Since phenotypically variant cells will typically have a deletion on one chromosome, the causative lesion can be limited to the homologous chromosome bounded by the deletion breakpoints. This portion of the genome can be sequenced to identify the causative mutation. In some variations, the use of insertional mutagens in combination with deletions allows precise localization and identification of disrupted genes causing the phenotypic variations. Alternatively, genomic DNA segments or expressed cDNAs can be introduced into the cells and analyzed for rescue of the altered phenotypes.

For purposes of clarification, unless the context clearly indicates otherwise, as used herein, "homologous chromosome," means a chromosome with the same, similar or comparable allele.

Described herein are methods allowing for direct functional phenotypic screening of a genome, and particularly a human genome, in cultured cells. This allows for direct testing in human cells rather than using model genetic organisms to approximate human biology. When the combination of mutagenesis methods described herein is used, genetic lesions can be more easily and cost-effectively mapped in cultured cells.
Types of Cells for Use in the Cell Culture-Based Methods Described Herein

Described herein are methods of screening cultured cells, including but not limited to cultured eukaryotic cells, for a recessive genotypic or phenotypic variation, mapping such variation to a region of a genetic sequence, and use of the information obtained thereby. Generally, any cell that can incorporate exogenous DNA into its genome can be analyzed by the methods described herein, including cells in primary tissues or cell lines. The methods described herein are broadly applicable to genetically manipulating many different types of organisms or cells, including but not limited to eukaryotic cells and organisms. Nonlimiting examples of cells and organisms for use in the methods described herein are those in the kingdoms protista, fungi, plantae, and animalia. Cells include both specialized differentiated cells, and pluripotent or totipotent cells.

In general, any cell or organism wherein the region to be analyzed is at least diploid has a "greater than haploid" genome, and may be used in the methods described herein. As used herein, "greater than haploid" may refer to multiples of the entire chromosome set, such as diploid, triploid, or tetraploid, multiples that deviate from an exact multiple of the haploid number of chromosomes, whether by having fewer (e.g., hypoploidy) or greater (e.g. hyperploidy) numbers of one or more chromosomes. As used herein, "greater than haploid" may also refer to a cell or organism with one or more additional copies of a given stretch of genetic sequence beyond what is native to a single chromosome. Non-limiting examples of a greater than haploid state include instances where a cell has greater than a single copy of a genetic sequence that is: (1) encoding a single gene, including its necessary regulatory elements, (2) a regulatory sequence, (3) a region wherein mutations are correlated with a disease state, or (4) a region that represents a part of a chromosome. The greater than single copy includes but is not limited to exogenous nucleic acid. In some variations, the exogenous nucleic acid is from a plasmid, virus, or any other exogenous source.

In some variations, a cell or organism that closely adheres to a diploid karyotype is used in the methods described herein. A cell or organism that closely adheres to a diploid karyotype is a cell or organism with two copies of each autosomal chromosome, with the normal complement of sex chromosomes for that species, with insubstantial or no partial chromosomal or whole chromosome deletions or duplications. As one nonlimiting example, in human cells a diploid karyotype corresponds to two copies of each autosomal chromosome and two sex chromosomes, for a total of 46 chromosomes, containing minimal or no partial chromosomal deletions or duplications.

In some variations, cells are described herein as "progenitor cells." A progenitor is a cell that was a precursor to a given cell. By way of non-limiting example, a cell having a recombinase-induced deletion and a further mutation may have 3 or more precursor cells: a precursor that had neither the recombinase-induced deletion nor the further mutation, a precursor that had the recombinase-induced deletion but not the further mutation, or a precursor that had the further mutation but not the recombinase-induced deletion.

Protists are a collective grouping of eukaryotic cells not having the characteristics of fungi, plants, or animals. Protists' life cycles consist of alternating haploid and diploid stages. The methods described herein are particularly relevant to manipulating the diploid stage of protists. Human disease-causing protists including *Giardia lamblia, Toxoplasma gondii, Trypanosoma cruzi,* and *Plasmodium* are non-limiting examples of protists that can be genetically manipulated by the methods described herein.

Fungi are eukaryotes whose life cycles consist of haploid, dikaryotic, and diploid stages. In some variations, the diploid fungal stage can be genetically manipulated by the methods described herein. Human disease is caused by infection of fungi of various genera, including but not limited to *Trichophyton, Sporothrix, Cladosporium, Phialophora, Fonsecaea, Epidermophyton, Microsporum, Piedraia, Trichosporon, Malassezia, Exophiala, Coccidioides, Fusarium, Penicillium*, and *Blastomyces.* Disease in immunocompromised human patients is also caused by opportunistic fungi of several genera, including, but not limited to, *Candida, Aspergillus, Zygomyces, Cryptococcus, Coccidioides, Histoplasma, Rhizopus, Mucor,* and *Absidia.*

Numerous fungal pathogens affect commercial food crops, for example by reducing yields and contaminating crops. Powdery mildews and rusts are fungi that commonly affect plant and flower cultivation. Other fungi are valued by humans. Non-limiting examples of commercially valuable mold include but are not limited to molds such as *Pencillium* species used to produce cheeses, *Rhizopus* species used to ferment soy products, and *Tolypocladium inflatum* as a source for the immunosuppressant cyclosporin. Yeasts such as *Saccromyces cerevisiae* are used in various culinary endeavors, including but not limited to the fermentation of wine and beer, and in bread making. Fungi such as mushrooms, truffles, and com fungus (*Ustilago maydis*) are consumable. Further, some fungi can catabolize pollutants as fuel sources, or convert undesirable contaminants to benign or more benign side-products. These and other fungi can be manipulated and analyzed using the methods described herein.

Plants are multicellular eukaryotes that are photosynthetic autotrophs. Plants include nonvascular and vascular plants, seedless and seed plants, and gymnosperms (conifers, cycads, etc.) and angiosperms (orchids, roses, tulips, etc.), and monocots and dicots. In some variations, the plants of agricultural significance, including but not limited to monocots, rice, wheat, corn, barley, buckwheat, millet, oats, rye, quinoa, spelt, wild rice, etc., legumes (black beans, pinto beans, kidney beans, soy beans, garbanzo beans, lentils etc.), nuts (almonds, hazelnuts, peanuts, chestnuts, pecans, walnuts, etc.), fruits (oranges, strawberries, bananas, grapes, avocados, tomatoes, squashes, etc.), tubers (potato, sweet potato, yarn, taro, jicama, etc), root vegetables (beets, carrots, radishes, etc), bulbs (onions, garlic, shallots, etc.), leaves (lettuce, spinach, watercress, arugula, cabbages), chili peppers, vegetables, grasses, cotton, hemp, sugarcane, tea, coffee, cocoa, tobacco, sunflower, safflower, canola, rapeseed, olive, coconut, palm, oak, mahogany, maple, redwood, and other evergreen and deciduous trees. Horticultural and ornamental plants species are also susceptible to manipulation and analysis by the method described herein. These and other plants can be manipulated with the methods described herein. Because plants can also be reproduced by several methods of vegetative reproduction or cloned on a large scale, plants are ideally suited for the methods described herein.

Animals are multicellular, heterotrophic eukaryotes. Since the diploid stage of many animals dominates the life cycle, animals are ideally suited for genetic manipulation with the methods described herein. Primary tissues or cells, and in some variations immortalized or transformed cell lines, may be analyzed by the methods described herein. In some variations the animals analyzed are animal cells corresponding to vertebrates, including but not limited to mammals (humans, primates, rodents, cows, etc.), birds (chickens, turkeys, etc.), reptiles (iguana, turtles, etc.), and amphibians (frogs, salamanders, newts, etc.). In some variations, primary cells or cell lines from animals are from major organs or glands (heart, lung, skin, stomach, brain, pancreas, testes, ovaries, breast, prostate, intestine, pituitary, adrenal gland, etc.) or any germ layer (ectoderm, endoderm, or mesoderm). Animal cells types susceptible to manipulation and analysis by the method described herein include, but are not limited to, endothelial cells, epithelial cells, neuronal cells, islet cells, mesenchymal cells, exocrine cells, endocrine cells, fibroblasts, mesothelial cells, mesodermal cells: Animal cells susceptible to manipulation and analysis by the method described herein are not limited to terminally differentiated cell types, but include pluripotent or totipotent stem cells, including but not limited to, embryonic stem cells, fetal germ cells, umbilical cord stem cells, adult stem cells, hematopoetic stem cells, neuronal stem cells, mesenchymal stem cells, olfactory stem cells, teeth stem cells, intestinal stem cells, germ line stem cells, wherein the cells are not human embryonic cells and not human germ line cells.

The methods described herein may be used, for example, in totipotent or pluripotent cells. In some variations, the methods described herein are used in stem cells, such as ES cells. In some variations, stem cells are differentiated into one or more cell types to analyze cell specific phenotypic variants or developmental potential. In some variations, mouse ES cells are used for large scale genetic screens in the ES cells or their derivatives. Additionally, mutant mouse ES cells can be transmitted through the germ line, which enables the study of the mutations in the animal. This feature alone can advance mouse genetics to a point where saturation genetics can performed in the ES cells from many genetic screens.

### Cell Culture-Based Screening

As described herein, there are many advantages to screening cultured cells rather than whole organisms. The methods of preparing cells and the screening methods described herein may generally be used to identify and map any recessive genetic or phenotypic variation.

FIG. 1 depicts some variations of methods of making deletions and mutations in chromosomes. In some variations, deletions that remove entire regions of a chromosome are exploited in genetic screens. FIG. 1-A depicts the result of treating cells such as eukaryotic cells or organisms with electromagnetic radiation, creating deletions in a random manner throughout the genome. As in all of the figures the top line represents a portion of one chromosome, and the bottom line represents the same portion of the homologous chromosome. The region indicated between the parentheses is the portion of the chromosome that is deleted.

FIG. 1-B depicts some variations of the result of treating cells with mutagens, including but not limited to chemical mutagens, or inhibitors of DNA repair. The asterisk (*) marks the location of a lesion in the DNA of a single chromosome.

FIG. 1-C depicts some variations of the result of treating cells with insertional mutagens (14). Insertional mutagens physically insert themselves into the DNA of chromosomes, sometimes disrupting an enhancer, a promoter, an untranslated region, or a coding region of a gene. Insertional mutagens include but are not limited to transposons, retrotransposons, retroviruses, pro-viral constructs, transgenes, plasmids, repetitive sequence elements, and gene traps.

FIG. 1-D depicts some variations for creating deletions that can be used to screen for phenotypic variants in cell culture. The targeting of positive (12) and negative (16) selectable markers to a defined genetic locus can allow a selection process for its deletion, as described in U.S. Patent 5,994,620 The targeted cells can be treated with electromagnetic radiation and deletions can be selected for by drug treatment against the presence of the negative selectable marker (FIG. 1-E).

FIG. 1-F depicts some variations of the use of gene targeting using homologous sequences both 5' and 3' to a positive selectable marker (12). Recombination occurs at the homologous sequences, indicated by the crossed lines. In this manner small regions of the chromosome can be deleted, as seen in FIG. 1-G by the region between the parentheses. Conventional gene targeting generally creates deletions only as large as one gene, and the other chromosome has a wild type allele. In order to create larger deletions, a chromosome can be targeted twice to place two site-specific recombination sites (10) in the same orientation (FIG. 1-H). Through conventional gene targeting a single chromosome can be targeted twice at predetermined loci, as described in U.S. Patent 4,959,317. Alternatively, the site-specific recombination sites (10) can be targeted at random as described in U.S. Patent 6,395,487 Either method provides for the opportunity to induce deletions, as seen in FIG. 1-I, by introducing a site-specific recombinase into these cells to catalyze the deletion.

One limitation to all of these methods is that each typically creates a lesion in only one chromosome, while the other chromosome is wild type and intact at every locus. Since recessive phenotypes are caused by the loss or reduction of gene products, or their functionality, from loci at both alleles of a gene, the methods of FIG. 1 alone cannot easily be used to directly screen for recessive phenotypes in cells. Traditionally, then, many of these methods are used in ES cells in order to make adult animals, or they are induced directly in animals. Through breeding, the lesions can be made homozygous. Animals with chromosomal deletions are commonly bred to other mutagenized animals to screen for phenotypic variation of the resulting offspring. This process is time consuming and costly.

By combining deletions of chromosomal DNA or nucleic acid sequences with, for example, additional deletions, chemical mutagenesis, insertional mutagenesis, methods affecting RNA or protein abundances or activities, or drug treatment, genotypically or phenotypically variant cells can be distinguished from parental cells absent the deletion or the mutation. In some variations, a deletion-generating method is combined with various methods of introducing mutations to generate mutations of all types, as opposed to using a single method that generates mutations of primarily one type. For example, mutagens that frequently cause point mutations are likely to induce certain types of mutations that gene traps will not induce, and vice versa. In some variations, a cell library comprises cells comprising an introduced deletion and further comprising an introduced deletion or mutation, wherein different clones in the cell library were generated using different methods of one or both of the deletion or mutagenesis methods.

Described herein are methods to create deletions within a desired region of the genome by introducing DNA into an organism or cell, including but not limited to eukaryotic organisms or cells, by one of a wide variety of methods known in the art. Methods of introducing DNA into cells include but are not limited to electroporation, microinjection, microparticle bombardment, viral or retroviral infection or transduction, transposition, retrotransposition, and transfection by means of DNA precipitation, lipofection, cationic lipid complexes, naked DNA transfection, or administration with a carrier agent including but not limited to polyethylene glycol (PEG). Following the introduction of DNA into an organism or cell, the insertion of the DNA into the genome is referred to as a targeted chromosome. The insertion point can be predetermined, for example in the case of homologous recombination, or random, for example, in the case of non-homologous insertion. In some variations, homologous recombination is used to target DNA to predetermined sites. In some variations, random insertion is used. In some variations, a combination of homologous recombination of DNA insertion and non-homologous insertion of a second DNA is used.

FIG. 2 illustrates some variations of the methods described herein. In FIG. 2-A one chromosome is consecutively targeted to insert two site-specific recombination sites (10) in the same orientation. A site-specific recombinase is introduced into the cells in order to confirm that the deletion can be made (FIG. 2-B). The deletion may be detected. The parental cell clone (FIG. 2-A) may be treated with chemical mutagens or inhibitors of DNA repair to induce mutations at random, indicated by an asterisk (FIG. 2-C). Then a site-specific recombinase may be introduced into the cells to create a cell clone with a deletion and a mutagen induced mutation (FIG. 2-D). Clones of cells in FIG. 2-D can be compared to parental cell clones in FIG. 2-C or FIG. 2-A for phenotypic variation.

Since deleted regions produced in some variations of FIG. 2-D can be extensive, comparative sequencing of genomic regions in FIG. 2-D and FIG. 2-A can be costly. The methods described herein may be used to easily and rapidly narrow the region of the genome to be comparatively sequenced to a degree that is cost effective.

Another non-limiting variation is illustrated in FIG. 6. In FIG. 6-B insertional mutagens (14) are used instead of chemical mutagens. Subsequent deletion of a part of the homologous chromosome in FIG. 6-C allows for the screening of recessive mutations in the cells.

Additional non-limiting variations are illustrated in FIG. 7. Cells with targeted chromosomes containing two site-specific recombination sites (10) in the correct orientation (FIG. 7-A), can be treated with site-specific recombinases to cause genomic deletions (FIG. 7-B). In some variations, deletion-containing cells are genetically sensitized substrates, and are used for screens involving drug treatment or methods to affect RNA or protein abundances or activities. Since the deletion-containing cells contain only half of the genetic complements for certain genes relative to the wild type cells, drug treatment, or methods to affect RNA or protein abundances or activities, can be more effective in screening for phenotypic variation. Methods to affect RNA or protein abundances or activities include but are not limited to RNA interference, antisense nucleic acids, DNAzymes and ribozymes.

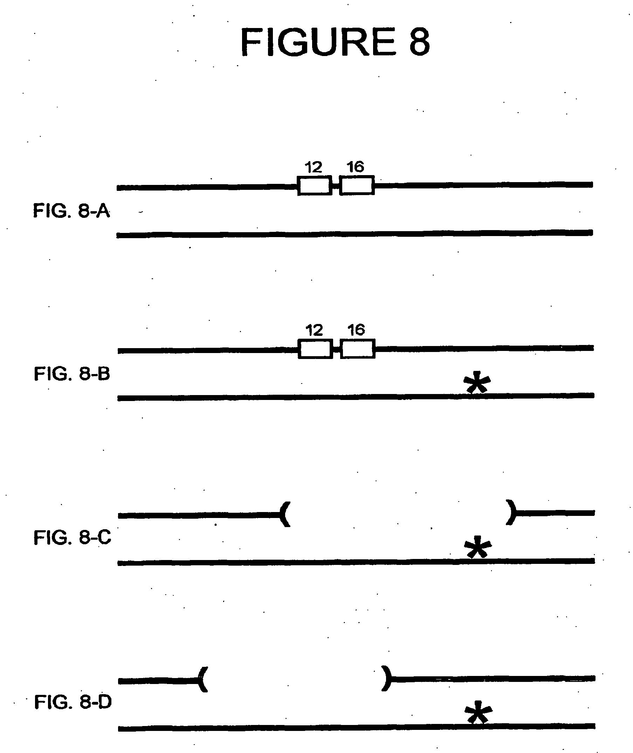

Also described herein are methods to induce deletions by electromagnetic radiation treatment combined with mutagen treatment, including but not limited to an insertional mutagen, in order to identify genetic lesions correlated with a genotypic or phenotypic variation. A method described in U.S. Patent 5,994,620, can be combined with mutagen treatment to comparatively screen cells or cell cultures instead of screening in adult mice. In some variations, the induced deletions are combined with the induced mutations in cells, and the cells are screened directly for phenotypic variation. In some variations, cells with positive and negative selectable markers (FIG. 8-A) are treated with mutagens or inhibitors of DNA repair (FIG. 8-B). Since the method creates nested deletions, cells that are homozygous mutant (FIG. 8-C) may show phenotypic variation, while cells with deletions that do not overlap the mutations on the homologous chromosomes (FIG. 8-D) may appear as wild type. Mapping the deletion breakpoints in these cells will narrow the region that contains the mutation.

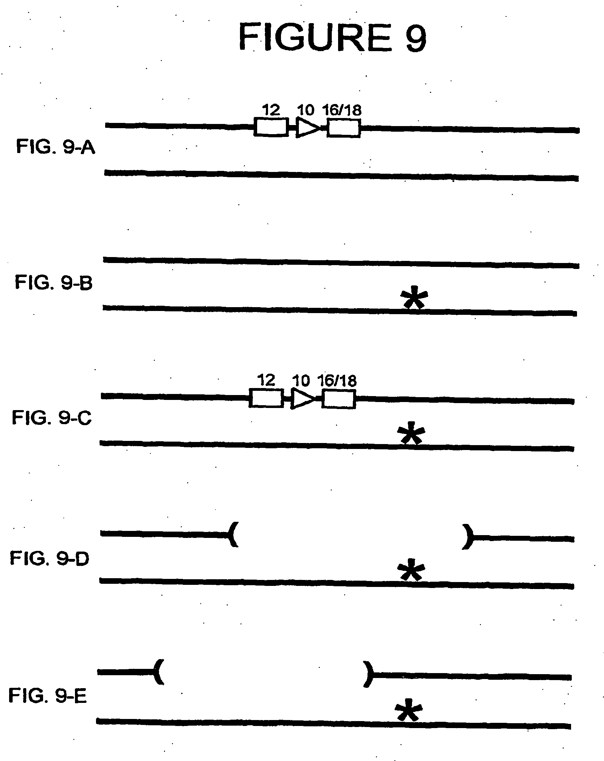

FIG. 9 illustrates some variations of the methods described herein. In FIG. 9-A, DNA containing a positive selectable marker (12), a site-specific recombination site (10), and a negative selectable marker (16) are introduced into the cells. In some variations DNA containing a positive selectable marker (12), a site-specific recombination site (10), and a visible marker (18) are introduced into the cells (FIG. 9-A). In some variations the introduced DNA can be flanked by a sequence homologous to a sequence in a cell, and cells can be selected for homologous recombination. In this variation, one advantage is in knowing the specific locus where the introduced DNA integrates. In some variations the DNA integrates randomly into the genome. In this variation, one advantage is the capacity to rapidly generate a plurality of cell clones, each containing an independent integration site into the genome.

In some variations, integration sites of the introduced DNA may be determined by various methods known by those of skill in the art, including but not limited to by splinkerette PCR, inverse PCR, or reverse transcriptase PCR. Cell clones may be treated with mutagens or inhibitors of DNA repair to induce mutations at random, indicated by an asterisk. In some variations the DNA construct is introduced into cell clones first (FIG. 9-A) and then treated with mutagens to induce random mutations (FIG. 9-C). In some variations cell clones are treated with mutagens to induce random mutations (FIG. 9-B) before the DNA construct is introduced into the cells (FIG. 9-C). Cells containing both induced mutations from mutagens and the introduced DNA construct (FIG. 9-C) may be treated with a deletion-inducing treatment, including but not limited to electromagnetic radiation, to induce genomic deletions (FIGS. 9-D, 9-E). In some variations, cell clones that contained an integrated negative selectable marker (16) may be selected for deletions at the site of integration, because surviving cell clones may have deleted the negative selectable marker. In some variations, the negative selectable marker confers sensitivity to a chemical or reagent. In some variations cell clones that contained an integrated visible marker (18) may be screened for the deletions at the site of integration by screening for the absence of the visible marker (18). Cell clones containing both induced deletions from electromagnetic radiation treatment and induced mutations from mutagen treatment (FIGS. 9-D, 9-E) may be compared to parental cell clones with no manipulations, to parental cell clones with an integrated DNA construct (FIG. 9-A), or to parental cell clones with induced mutations (FIG. 9-B) for genotypic or phenotypic variation. In some variations, cell clones that show genotypic or phenotypic variation have a deletion on one chromosome that uncovers a mutation (*) on the homologous chromosome (FIG. 9-D). In some variations, cell clones that have a deletion on a portion of one chromosome that does not uncover a mutation (*) on the homologous chromosome (FIG. 9-E) may not show genotypic or phenotypic variation.

In some variations, the methods described herein may be used with cells comprising a deletion in each of two homologous chromosomes. In some variations, at least one of the deletions is introduced. In some variations, two or more deletions are introduced in two or more homologous chromosomes. In some variations, a second deletion is introduced into a cell or population of cells comprising a deletion, wherein the first deletion occurred naturally. In some variations, a second deletion is introduced into a cell or population of cells comprising a deletion, wherein the first deletion was previously introduced.

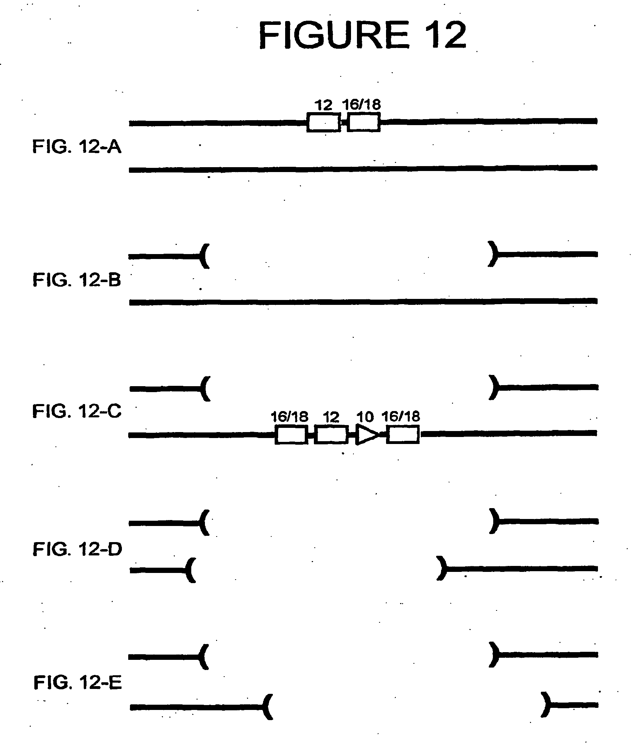

FIG. 12 illustrates some variations of the methods described herein. In FIG. 12-A, DNA containing a positive selectable marker (12) and a negative selectable marker (16) are introduced into the cells. In some variations DNA containing a positive selectable marker (12), a site-specific recombination site (10), and a negative selectable marker (16) are introduced into the cells. In some variations cells containing the markers are treated with electromagnetic radiation and deletions at the point of integration are selected for by drug treatment against the inclusion of the negative selectable marker (FIG. 12-B). In some variations deletion containing cells (FIG. 12-B) are introduced with DNA containing a positive selectable marker (12), a site-specific recombination site (10), and a negative selectable marker (16) or a visible marker (18) (FIG. 12-C). In some variations the integration is by homologous recombination. In some variations cells containing a deletion and a negative selectable marker in the corresponding homologous chromosome segment (FIG. 12-C) are treated with electromagnetic radiation and deletions at the integration site are selected for by drug treatment against the negative selectable marker (FIGS. 12-D, 12-E). In some variations cells containing heteroallelic deletions are screened for phenotypic or genotypic variation.

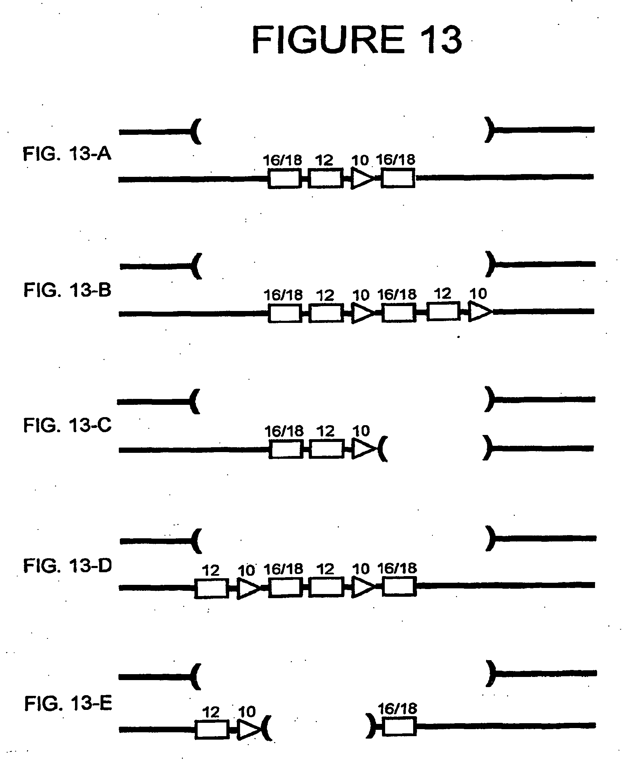

FIG. 13 illustrates some variations for localizing genetic lesions conferring phenotypic or genotypic variance. In some variations, cells containing a deletion in one allele and a site-specific recombination site on the corresponding homologous chromosome segment (FIG. 13A) are introduced with DNA containing a site-specific recombination site (10) and a positive selectable marker (12). Site-specific recombinase may be introduced to cells containing two site-specific recombination sites (10) (FIGS. 13-B, 13-D), resulting in smaller heteroallelic deletions (FIG. 13-C, 13-E). In some variations, cell clones that have a heteroallelic deletion combination in a chromosomal segment (FIG. 13-C) may show phenotypic variation, while cell clones that have a heteroallelic deletion combination in a neighboring chromosomal segment (FIG. 13-E) will not show phenotypic variation. The process illustrated in FIG. 13 may be reiterated to narrow the region containing the heteroallelic deletion, to determine the genetic sequence that is correlated with the phenotypic variation.

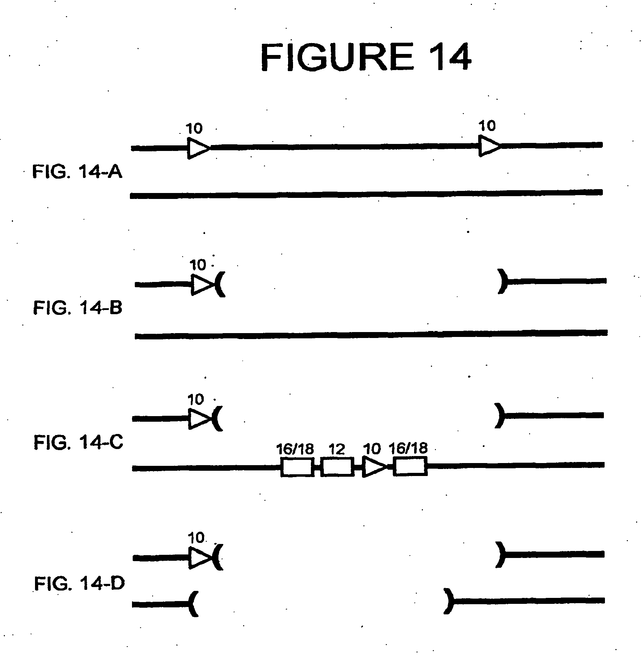

FIG. 14 illustrates some variations of the methods described herein. In FIG. 14-A one chromosome is consecutively targeted to insert two site-specific recombination sites (10) in the same orientation. A site-specific recombinase is introduced into the cells in order to induce a deletion (FIG. 14-B). In some variations deletion containing cells (FIG. 14-B) are introduced with DNA containing a positive selectable marker (12), a site-specific recombination site (10), and a negative selectable marker (16) or a visible marker (18) (FIG. 14-C). In some variations the integration is selected for by homologous recombination. In some variations cells containing a deletion and a negative selectable marker in the corresponding homologous chromosome segment (FIG. 14-C) are treated with electromagnetic radiation and deletions at the integration site are selected for by drug treatment against the negative selectable marker (FIGS. 14-D). In some variations cells containing a deletion and a visible marker are treated with electromagnetic radiation and deletions at the integration site are selected for by the absence of the visible marker (FIGS. 14-D). In some variations cells containing heteroallelic deletions are screened for phenotypic or genotypic variation.

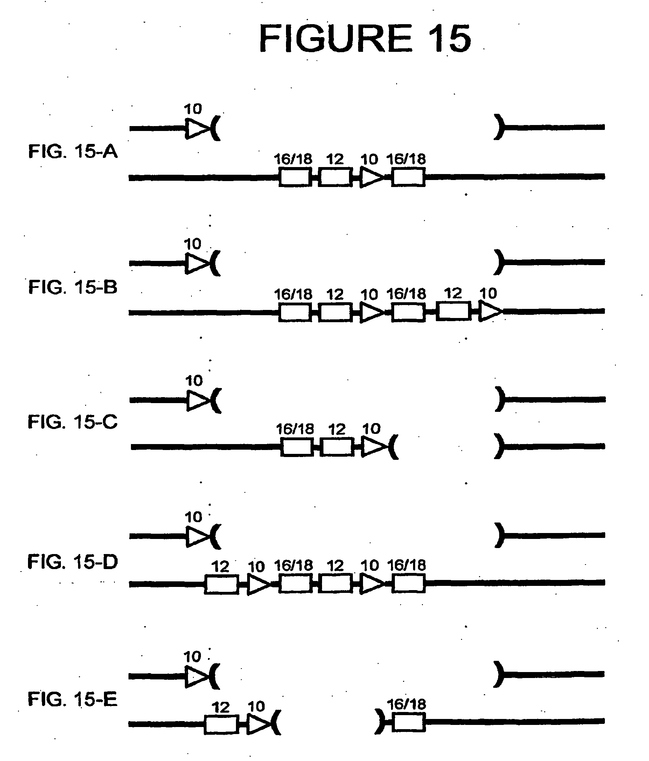

FIG. 15 illustrates some variations for localizing genetic lesions conferring phenotypic or genotypic variance. Phenotypic variant cells containing heteroallelic deletions (FIG. 14-D) are derived from cells containing a deletion and a site-specific recombination site on the corresponding homologous chromosome segment (FIG. 15-A). These cells are introduced with DNA containing a positive selectable marker (12) and a site-specific recombination site (10) (FIGS. 15-B, 15-D). In some variations site-specific recombinase is introduced to cells containing two site-specific recombination sites (10) in the same orientation (FIGS. 15-B, 15-D), resulting in smaller heteroallelic deletions (FIGS. 15-C, 15-E). In some variations, cell clones that have a heteroallelic deletion combination in a chromosomal segment (FIG. 15-C) may show phenotypic variation, while cell clones that have a heteroallelic deletion combination in a neighboring chromosomal segment (FIG. 15-E) will not show phenotypic variation. The process illustrated in FIG. 15 may be reiterated to narrow the region containing the heteroallelic deletion, to determine the genetic sequence that is correlated with the phenotypic variation.

In some variations a first deletion is induced by exposing cells to deletion causing mutagens, including but not limited to electromagnetic radiation. In some variations, genomic comparison between the parental cell clones and the daughter cell clones is used to determine the location of the induced deletion. Genomic comparison methods include but are not limited to comparative genomic hybridization, fluorescence in situ hybridization, multiplex fluorescence in situ hybridization, spectral karyotyping, Giemsa stain karyotyping, Southern blot, and gene and genomic array hybridization.

In some variations a second deletion is induced after introducing DNA containing positive and negative selectable markers that will be (a) targeted to the region corresponding to the deletion on the homologous chromosome, or (b) inserted randomly. Cells are then be treated with electromagnetic radiation to induce deletions, and drugs or other stimuli are added to select against the negative selectable marker. Surviving cells are likely to have chromosomal deletions at the integration site of the negative selectable marker.

In some variations a second deletion is induced after one chromosome is consecutively targeted to insert two site-specific recombination sites in the same orientation in the general region corresponding to the deletion on the homologous chromosome. A site-specific recombinase is introduced into the cells in order to induce the second deletion.

In some variations the order of the deletions is reversed. In some variations, a first deletion is induced by treating cells containing inserted positive and negative selectable markers with electromagnetic radiation, and selecting with drugs against the negative selectable marker. In some variations, a first deletion is induced by introducing a site-specific recombinase to cells containing two site-specific recombination sites in the same orientation on one chromosome. In some variations, a second deletion is induced by treating the deletion containing cells with deletion causing mutagens, including but not limited to electromagnetic radiation.

In some variations, cell clones containing heteroallelic deletions may be screened for genotypic or phenotypic variation.

### Detailed Discussion of Cells and Cell Culture-Based Screening

Below are nonlimiting examples of screening methods that may be used to identify recessive genotypic or phenotypic variations; unless the context clearly indicates otherwise, the genetic sequences associated with the recessive genotypic or phenotypic variation may be mapped using any of the methods described herein.

In some variations, cells can be screened for their response to various pathogens, including but not limited to viral or bacterial pathogens, including but not limited to HIV, Rhinoviruses, Influenza, or Respiratory Syncitial Virus infecting tracheal or lung epithelium, and Epstein-Barr virus infecting B-cells. In some variations, genetically manipulated human T cell lines expressing the correct viral entry receptors and co-receptors are infected in cell culture with Human Immunodeficiency Virus (HIV). In a further variation, such cell clones can be screened for the ability of HIV to enter, infect, replicate, kill, exit, or shed in cells. Cell clones in which HIV fails to enter, infect, replicate, kill, exit, or shed in the cells are likely to contain mutations in human genes that are necessary for its viral life cycle. In some variations, these host genes are targets for, for example, inhibition. This may provide therapies for HIV.

Some human cancers are characterized by one or more of unrestrained growth, invasive growth into other tissues, recruitment of vasculature, and disseminated growth by metastases. In some variations, by genetically manipulating cancer cell lines with the methods described herein, clones can be screened for the suppression of cancer cell phenotypes both in cell culture and when injected into animals. In some variations, cancer cells are screened for increased sensitivity to chemotherapeutic drugs. Sensitive cell clones are likely to contain mutations in human genes that confer resistance to small molecule drugs (e.g. protein pumps or channels that eliminate cytotoxic molecules). These genes are targets for inhibition, which can increase the potency of chemotherapeutic drugs, which in turn can lead to decreased toxicity and equal potency at lower doses. When combined with inhibitors to these genes, human patients could receive more effective doses of chemotherapeutic drugs while lessening nausea and other side effects. In some variations, cancer cells are screened for their ability to form colonies in soft agar. Cancer cell clones that show decreased ability to grow in soft agar are likely to contain mutations in genes that allow for unrestrained growth. These genes are targets for inhibition, which can limit the ability of cancer cells to grow unrestrained in patients. In some variations, human cancer cells are xenografted in experimental animals such as severe combined immunodeficient (SCID) mice. In some variations, human cancer cells xenographed in ectopic or orthotopic locations in experimental animals are visualized for growth, invasive growth into other tissues, recruitment of vasculature, and disseminated growth by metastases. Cancer cell clones that show a suppression of any of these cancer phenotypes are likely to contain mutations in human genes that are necessary for cancer progression. These genes and their downstream effector molecules are targets for inhibition or alteration, which may provide therapies for cancer. Similarly, any other known genotypic or phenotypic variation of cells with a cancer-like genotype or phenotype may be analyzed using the methods described herein.

Alzheimer's disease is associated with plaques of a protein called β -amyloid. Specific neuronal cell types produce the processed protein β-amyloid, which is thought to contribute directly to disease progression. Cell clones expressing β-amyloid precursor protein (at physiological levels or higher, or at elevated levels from expression constructs) can be screened for the suppression of β-amyloid production directly in cell culture. Cell clones that no longer express or process the amyloid precursor protein, produce or secrete β-amyloid, or die as a result of β-amyloid accumulation, are likely to contain mutations in human genes that are necessary for the formation of β-amyloid plaques. These genes are targets for inhibition or alteration which may provide therapies for Alzheimer's disease. The same strategy could be used to examine the involvement of tau protein in the plaques of Alzheimer's disease. Similarly, any other known genotypic or phenotypic variation of cells with an Alzheimer's-like genotype or phenotype may be analyzed using the methods described herein.

Parkinson's disease is associated with plaques of a protein called α-synuclein. Specific neuronal cell types produce the process protein α-synuclein, which is thought to contribute directly to disease progression. Cell clones expressing α-synuclein (at physiological levels or higher, or at elevated levels caused by expression constructs) can be screened for the suppression of α-synuclein production directly in cell culture. Cell clones that no longer express, secrete, or die as a result of α-synuclein accumulation, are likely to contain mutations in human genes that are necessary for the formation of α-synuclein plaques. These genes and their downstream effector molecules are targets for inhibition or alteration, which may provide therapies for Parkinson's disease. Similarly, any other known genotypic or phenotypic variation of cells with a Parkinson's-like genotype or phenotype may be analyzed using the methods described herein.

Osteoporosis is characterized, in part, by a gradual decline in bone mass and density that makes affected patients more susceptible to bone breaks. Bone is catabolized by cells termed osteoclasts. Bone is anabolized by cells termed osteoblasts. Genetically manipulated osteoclast and osteoblast cell clones can be screened for enhancement or suppression of function. Cell clones that suppress osteoclast function or enhance osteoblast function, are likely to contain mutations in human genes that are necessary for their function in bone metabolism. These genes are targets for inhibition, which may provide therapies for osteoporosis. Similarly, any other known genotypic or phenotypic variation of cells with an osteoporosis-like genotype or phenotype may be analyzed using the methods described herein.

The methods described herein are not confined to human or animal cells. The methods described herein can manipulate the genome of any organism with a greater than haploid genome. The method of introducing site-specific recombination sites onto chromosomes may vary from one species to another, but the method of creating genomic deletions in combination with mutations can be used in any cell with a greater than haploid genome, including but not limited to diploid genomes.

Protists including *Giardia lamblia, Toxoplasma gondii, Trypanosoma cruzi,* and those belonging to the genus *Plasmodium* are the cause of the respective human diseases, diarrheal Giardiasis, toxoplasmosis, Chagas' disease, and malaria. In some variations, during the diploid stages of the protists life cycle, or aneuploid in the case of *Trypanosoma cruzi,* the protists are manipulated by the methods described herein. Protists can be screened for their inability to undergo or complete meiosis, or transition to other stages of their life cycle. Protists that cannot continue in their life cycle are likely to contain mutations that disrupt essential cellular processes. These genes and their downstream effector molecules are targets for inhibition or alteration, which may lead to therapies preventing the viability of human disease-causing protists.

Human disease is caused by infection of fungal pathogens from several genera including, but not limited to, *Trichophyton, Sporothrix, Cladosporium, Phialophora, Fonsecaea, Epidermophyton, Microsporum, Piedraia, Trichosporon, Malassezia, Exophiala, Coccidioides, Fusarium, Penicillium, Blastomyces, Candida, Aspergillus, Zygomyces, Cryptococcus, Coccidioides, Histoplasma, Rhizopus, Mucor, and Absidia.* These fungal pathogens have diploid or dikaryotic stages of their life cycle that can be manipulated by the methods described herein. Fungi that cannot undergo or complete meiosis, sporulation, or other stages in their life cycle are likely to mutations that disrupt essential cellular processes. These genes and their downstream effector molecules are targets for inhibition or alteration, which may lead to therapies preventing the viability of human disease-causing fungi.

Mutated fungi can also be screened for desirable characteristics, and the genetic basis for such characteristic can be transferred to other fungi or organisms.

Some types of fungi are plant pathogens. These fungi cause crop losses, threaten food security, and can pose dangerous risks to humans when fungi produce mycotoxins in infected crops. Fungi also affect the health of commercial and ornamental flowering plants, trees, and grasses. These fungal pathogens have diploid or dikaryotic stages of their life cycle that can be manipulated by the methods described herein. Leaf disk and in vitro assays can be used to screen for the suppression of growth of these fungal pathogens. Fungi that cannot grow on plant tissues are likely to contain mutations that disrupt essential cellular processes. These genes are targets for inhibition, which may lead to treatments preventing the viability and growth of plant disease-causing fungi.

Plant cell protoplasts can be manipulated by the methods described herein, and resulting plants can be screened for any desired phenotypic variant. Phenotypes may include, but are not limited to, growth rate, growth in various soil conditions (e.g. high salt or metal concentration, extreme pH or temperature), growth under water deficit, flooding, or chilling stresses, altered fruit size or taste, enhanced nutritional value, flower size or color, ripening rate, fiber strength or color. Characteristics may be transferred from one species of plant to another. For example, the methods described herein allow for the identification of genes needed to produce a pigment in one species of plant. That gene or genes can then be transgenically expressed in another plant, such as by cotton fiber-specific promoters to produce pigmented cotton. Legumes have root nodules that maintain a symbiotic relationship with *Rhizobia* bacteria, resulting in the fixation of nitrogen. The methods described herein allow legumes to be genetically manipulated to determine the genes necessary for one or more of *Rhizobia* bacterial infection, formation of the infection thread, production of hemoglobin, and formation of root nodules. These genes can be transgenically expressed in the roots of non-legume plants to fix nitrogen in other commercial crops.

The genotypic and phenotypic variation is not limited to the examples described herein. Persons skilled in the art have the ability to search for any cellular genotype or phenotype with the methods described herein.

Clones that show phenotypic variation can be tested for the dependence upon the deletion or mutation. In some variations, each deletion-containing clone is derived from a cloned cell that was mutagenized. In this variation, the parental clone contains all of the same induced mutations, but does not have the induced deletion. This parental clone can be tested for the same phenotypic variation; the absence of a phenotype in the absence of the deletion indicates there exists a correlated mutation, including but not limited to recessive mutations, bounded by the deletion boundaries. In some variations, the DNA of the whole region is sequenced. In some variations, the region is first narrowed. In some variations, a third site-specific recombination site is targeted to the chromosome in the correct orientation in addition to or in between the previous two sites. Subsequent introduction of a site-specific recombinase allows for at least three different deletion possibilities as non-limiting illustrated in FIGS. 3-C, 3-D, 3-E. Cells in FIG. 3-C have a mutation on one chromosome, but the other allele on the homologous chromosome is intact and wild type, so the cells may not show phenotypic variation. Cells in FIGS. 3-D, 3-E have a mutation on one chromosome and the homologous chromosome is deleted at that locus, so these cells are more likely to show phenotypic differences. In some variations, the process illustrated in FIG. 3 is performed two or more times in parallel, or reiterated in series to narrow the region containing the mutation, to a point where it is cost effective or desirable to comparatively sequence the chromosomal region.

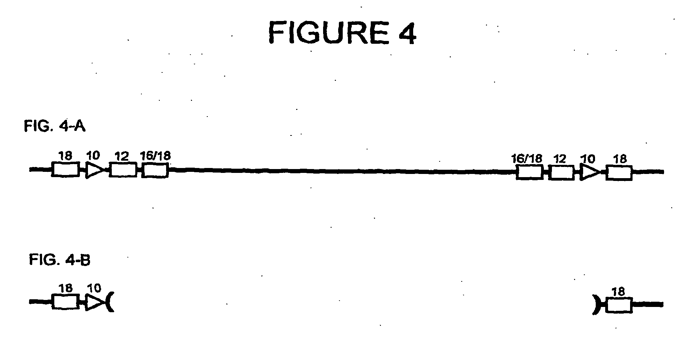

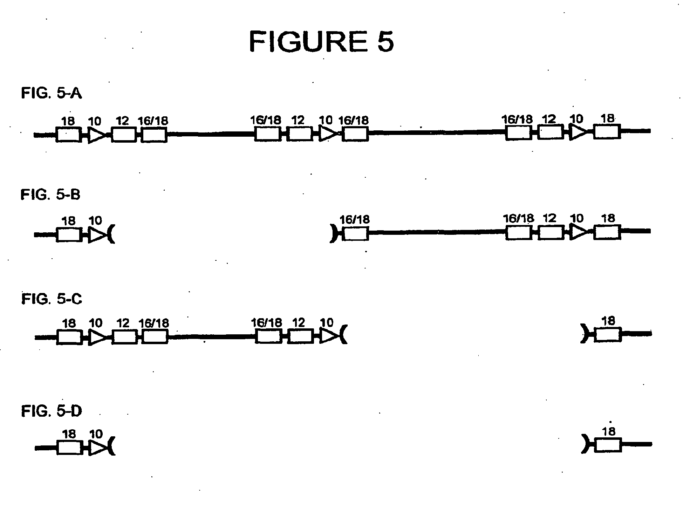

The types of deletions produced can be selected for directly by the use of, for example, drug selectable markers. In some variations depicted in FIG. 4-A, negative selectable markers exist between the site-specific recombination sites. In some variations, cells that delete the intervening sequence (FIG. 4-B) when the site-specific recombinase is added are unaffected by the drugs added to the media. Cells that retain the intervening sequence have negative selectable markers that, for example, convert drugs into toxic intermediates, resulting in cell death. In this manner, the minority of cells that create deletions can be directly selected. In order to narrow the deletion area to map the mutation on the homologous chromosome, a third site-specific recombination site can be targeted to the chromosome (FIG. 5-A). Since each positive and negative selectable marker can work independently, different combinations of drugs can select for desired deletions. In this manner, cells containing, for example, deletions seen in FIGS. 5-B, 5-C, 5-D can be specifically selected for when a site-specific recombinase is added to the cells.

In some variations, insertional mutagens are used to induce mutations. In some variations, cells containing site-specific recombination sites are treated with insertional mutagens (FIG. 6-A). Some insertions will integrate in chromosomal regions that are bounded by sites on the homologous chromosome (FIG. 6-B). Subsequent deletion can reveal recessive phenotypes (FIG. 6-C).

In some variations, the use of deletion-containing cells, with or without other induced mutations, to screen for phenotypic variants can be combined with drug treatment or methods affecting RNA or protein abundances or activities. In some variations, drug libraries are used to find specific inhibitors of gene products or cellular processes. Methods of affecting RNA or protein abundances or activities may be used to analyze specific inhibition of gene products on cellular processes. Wild type diploid cells have two functional gene copies on two homologous chromosomes. By deleting half of the gene dosage in a particular chromosomal segment, cells are genetically sensitized to additional perturbations. Therefore, drug screening or methods affecting RNA or protein abundances or activities can be more effective on deletion-containing cells than on wild type diploid cells.

In some variations, rather than using site-specific recombination sites and site-specific recombinase to produce deletions in cells, cells deletions are made at random using, for example, electromagnetic radiation treatment. In some variations, resulting cells are analyzed by the methods described herein and used to screen cells. In some variations, positive and negative selectable markers are targeted to chromosomes or inserted randomly (FIG. 1-D). Cells are then treated with electromagnetic radiation to induce deletions and drugs or other appropriate stimuli are added to select against the negative selectable marker. Surviving cells are likely to have chromosomal deletions at the integration site of the negative selectable marker (FIG. 1-E). In some variations, of the methods described herein, the induced deletions are combined with the induced mutations in the cells, and the cells are screened directly for phenotypic variation. Cells targeted with the positive and negative selectable markers (FIG. 8-A) are treated with mutagens or inhibitors of DNA repair (FIG. 8-B). In some variations, since the method creates nested deletions, cells that are homozygous mutant (FIG. 8-C) will show phenotypic variation, while cells with deletions that do not overlap the mutations on the homologous chromosomes (FIG. 8-D) are wild type. Mapping the deletion breakpoints in these cells will narrow the region that contains the mutation.

In some variations, cells are made competent to induce regional mutations. Cells are then treated with mutagens or inhibitors of DNA repair. Deletions are then induced in the cells. Cells are then tested for phenotypic variation. In some variations, deletions in cells are generated before inducing other mutations. While this may complicate mapping strategies described herein, comparative sequencing of the chromosomal region would identify mutations. Creating deletions before treating with mutagens or inhibitors of DNA repair would also save effort in inducing the deletions in each individual clone.

In some variations, positive and negative selectable markers are targeted to chromosomes or inserted randomly (FIGS. 1-D, 12-A). Cells are then treated with electromagnetic radiation to induce deletions and drugs or other appropriate stimuli are added to select against the negative selectable marker. Surviving cells are likely to have chromosomal deletions at the integration site of the negative selectable marker (FIGS. 1-E, 12-B). In some variations, of the methods described herein, positive and negative selectable markers are targeted to chromosomes or inserted randomly into cells containing the induced deletions (FIG. 12-C). Cells are then treated with electromagnetic radiation to induce deletions and drugs or other appropriate stimuli are added to select against the negative selectable marker. Surviving cells are likely to have chromosomal deletions at the integration site of the negative selectable marker (FIGS. 12-D, 12-E). In some variations cells containing heteroallelic deletions (FIGS. 12-D, 12-E) are screened for phenotypic variations. In some variations the deletion containing cells (FIGS. 12-B, 13-A) can be targeted with site-specific recombination sites (FIGS. 12-C, 13-A, 13-B, 13-D) and later introduced with a site-specific recombinase to cause deletions on the homologous chromosome (FIGS. 13-B, 13E). In some variations cells containing heteroallelic deletions (FIGS. 13-B, 13E) are screened for phenotypic variations.

In some variations, one chromosome is consecutively targeted to insert two site-specific recombination sites (10) in the same orientation (FIG. 14-A). A site-specific recombinase is introduced into the cells in order to induce a deletion (FIG. 14-B). In some variations deletion containing cells (FIG. 14-B) are introduced with DNA containing a positive selectable marker (12), a site-specific recombination site (10), and a negative selectable marker (16) or a visible marker (18) (FIG. 14-C). In some variations the integration is by homologous recombination. In some variations cells containing a deletion and a negative selectable marker in the corresponding homologous chromosome segment (FIG. 14-C) are treated with electromagnetic radiation and deletions at the integration site are selected for by drug treatment against the negative selectable marker (FIGS. 14-D). In some variations cells containing a deletion and a visible marker are treated with electromagnetic radiation and deletions at the integration site are selected for by the absence of the visible marker (FIGS. 14-D). In some variations cells containing heteroallelic deletions are screened for phenotypic or genotypic variation. In some variations phenotypically variant cells containing heteroallelic deletions (FIG. 14-D) are derived from cells containing a deletion and a site-specific recombination site on the corresponding homologous chromosome segment (FIG. 15-A). These cells are introduced with DNA containing a positive selectable marker (12) and a site-specific recombination site (10) (FIGS. 15-B, 15-D). In some variations site-specific recombinase is introduced to cells containing two site-specific recombination sites (10) in the same orientation (FIGS. 15-B, 15-D), resulting in smaller heteroallelic deletions (FIGS. 15-C, 15-E). In some variations, cell clones that have a heteroallelic deletion combination in a chromosomal segment (FIG. 15-C) may show phenotypic variation, while cell clones that have a heteroallelic deletion combination in a neighboring chromosomal segment (FIG. 15-E) will not show phenotypic variation. The process illustrated in FIG. 15 may be reiterated to narrow the region containing the heteroallelic deletion, to determine the genetic sequence that is correlated with the phenotypic variation.

The methods herein are described with particular emphasis towards mammalian cultured cells. However, any greater than haploid cell or organism, including but not limited to diploid eukaryotic cells or organisms, can be manipulated according to the methods described herein.

Non-limiting examples of cells that may be used in the methods described herein are those with one or more of the following characteristics: cells including but not limited to cancer cells that have a greater than haploid content, that contain a nearly diploid content, that grow colonies in semi-solid medium, that are resistant to various types of chemotherapy, that induce neo-angiogenesis, that grow when xenografted in experimental animals, or that may metastasize within experimental animals.

Cells that may be manipulated by the methods described herein include human lung cancer cell lines. Cancer cell lines frequently contain deletions or duplications of portions of chromosomes, or entire chromosome losses or gains. A cancer cell line that most closely adheres to a diploid karyotype is the ideal substrate, although any cell line with a greater than haploid karyotype can be utilized. In human cells a diploid karyotype corresponds to two copies of each autosomal chromosome and two sex chromosomes, for a total of 46 chromosomes, containing minimal or no partial chromosomal deletions or duplications. Human lung cancer cell lines that contain a mostly diploid karyotype include but are not limited to, DV90, HLF-a, H522, H460, H460SM, SCLC-R1, SCC-37, Ma-4, Ma-10, Ma-12, Ma-15, and Ia 162. An alternative to using a cell line derived from a cancer cell or a tumor is to transform a primary cell or cell line. Nonlimiting examples of ways to transform cells include but are not limited to continuous culturing, treatment of cell with glycidyl methacrylate or other mutagens, and the co-expression of the SV-40 large-T antigen, a telomerase catalytic subunit, and an H-Ras oncoprotein.

In some variations, lung cancer cell lines are individually cloned to isogenize the starting population of cells. Non-limiting examples of methods to individually clone cells include fluorescence activated cell sorting and limited dilution cell culturing. In some variations, aliquots of the isogenized cell clone are cryogenically preserved to maintain a parental population for genetic comparison to manipulated daughter cells. In some variations, DNA containing a first site-specific recombination site are introduced into isogenized cell clones. Site-specific recombination sites include but are not limited to loxP sites, loxP sites with variable spacer regions, FRT sites, attB sites, attP sites, attL sites, and attR sites.

The nonlimiting example of a Cre recombinase site is used in the following discussion; however, any site-specific recombination site may be used in the methods described herein.

Non-limiting examples of methods of introducing DNA into the cells include, but are not limited to, electroporation, microinjection, microparticle bombardment, viral or retroviral infection or transduction, transposition, retrotransposition, and transfection by means of DNA precipitation, lipofection, cationic lipid complexes, naked DNA transfection, or administration with a carrier agent including but not limited to polyethylene glycol (PEG).

In some variations the DNA is introduced into the isogenized cell clones for integration in a random manner, for example by way of random plasmid or transgene integration, proviral or viral constructs, transposon or retrotransposon constructs, or gene trap constructs. Variations with random integration of the first Cre recombinase site may have the advantage that large numbers of individual clones containing unique integration sites can be isolated easily. Methods to individually clone cells include but are not limited to fluorescently activated cell sorting and limited dilution cell culturing. Individual cell clones containing Cre recombination sites may be grown in sufficient quantities to cryogenically preserve aliquots and to extract both DNA and RNA from cell clones. In some variations the location of the random integration site into the host genome is identified by splinkerette PCR, inverse PCR, or reverse transcriptase PCR. In some variations, cell clones containing a single integration site of a Cre recombination site are used for further manipulation.

In the case of random integration of plasmids or transgenes, head to tail concatamers containing several copies of the introduced DNA construct are common. In some variations, the copy number can be reduced by introduction of the Cre recombinase. In some variations the copy number is reduced to one. Methods to introduce Cre recombinase include but are not limited to microinjection of Cre recombinase protein, electroporation, microinjection, microparticle bombardment, viral or retroviral infection or transduction, and transfection by means of DNA precipitation, lipofection, cationic lipid complexes, naked DNA transfection, or administration with a carrier agent including but not limited to polyethylene glycol (PEG) of a DNA construct containing a promoter expressing the Cre recombinase gene.

In some variations DNA is introduced into isogenized cell clones to integrate a Cre recombinase site in a predetermined site in the genome. In a nonlimiting example of targeting a site-specific recombinase site into the genome, homologous recombination may be used. In some variations the DNA vector to be targeted minimally contain a 5' flanking sequence homologous to endogenous genomic sequence, a Cre recombinase site, and 3' flanking sequence homologous to endogenous genomic sequence. In some variations a DNA vector with additional functional elements contains, in order, a 5' flanking sequence homologous to an endogenous genomic sequence, a visible marker such as a green fluorescent protein expression cassette, a Cre recombinase site, one or more selectable markers such as a neomycin resistance gene expression cassette or a herpes simplex virus thymidine kinase expression cassette, 3' flanking sequence homologous to endogenous genomic sequence, and an optional diptheria toxin or other negative selectable marker expression cassette. The diptheria toxin expression cassette or other negative selectable marker is optional, as it typically enriches homologous recombination events by only five- to ten-fold. In this variation, cell clones introduced with the DNA construct are selected for based on the selectable marker, for example by a neomycin analog such as G418. In a further variation, individual cell clones are isolated by fluorescent activated cell sorting or limited dilution cell culturing. In a further variation a plurality of cell clones are isolated and are grown in sufficient quantities to cryogenically preserve aliquots to extract DNA from cell clones. In a further variation, individual clones are tested for homologous recombination at the locus determined by the flanking homologous sequences within each individual vector. Methods to determine homologous recombination at each locus include but are not limited to Southern blots and polymerase chain reaction. Clones are identified as containing a Cre recombinase site integrated into the genome in a predetermined locus.

In some variations, whether using random integration or homologous recombination at a predetermined locus, cell clones containing a single Cre recombinase site are utilized for further genetic manipulation. In some variations, DNA containing a second Cre recombinase site are introduced into cell clones containing an integrated Cre recombinase site. In some variations, the DNA vector to be targeted minimally contains a 5' flanking sequence homologous to endogenous genomic sequence, a Cre recombinase site, and 3' flanking sequence homologous to endogenous genomic sequence. In some variations a nucleic acid vector with additional functional elements contains one or more of a 5' flanking sequence homologous to endogenous genomic sequence, a negative selectable marker such as a cytidine deaminase and uracil phosphoribosyltransferase fusion expression cassette, a hygromycin resistance gene expression cassette, a Cre recombinase site, a positive marker such as a β-galactosidase expression cassette, a 3' flanking sequence homologous to endogenous genomic sequence, and an optional diptheria toxin expression cassette. The diptheria toxin expression cassette or a comparable negative selectable marker is optional, as it typically enriches homologous recombination events by only five fold. In such a variation, cell clones introduced with the nucleic acid construct may be selected for based on the positive selectable marker, such as a hygromycin-containing medium. In some variations, individual cell clones are isolated by fluorescent activated cell sorting or limited dilution cell culturing. In some variations a plurality of cell clones is isolated and grown in sufficient quantities to cryogenically preserve aliquots to extract DNA from cell clones. In some variations individual clones are tested for homologous recombination at the locus determined by the flanking homologous sequences within each individual vector. Methods to determine homologous recombination at each locus include but are not limited to Southern blots and polymerase chain reaction. In some variations clones are identified as containing a second Cre recombinase site integrated into the genome in a known or predetermined locus.

In diploid cells, the second Cre recombinase site will have a 50% probability of integrating into the same chromosome containing the first Cre recombinase site, and a 50% probability of integrating into the homologous chromosome. Recombination at two Cre recombinase sites on the same chromosome in the same orientation may result in a deletion of the intervening sequence. Recombination at two Cre recombinase sites on the same chromosome in the opposite orientation may result in an inversion of the intervening sequence. Recombination at two Cre recombinase sites on homologous chromosomes may result in a reciprocal translocation.

In some variations, DNA containing an expression cassette of Cre recombinase is introduced into cell clones containing two Cre recombinase sites integrated into the genome at known loci. Cells clones containing deletions are enriched for by drug selection against negative selectable markers, for example by ganciclovir and 5-fluorocytosine drug selection against the inclusion of the herpes simplex virus thymidine kinase expression cassette and cytidine deaminase and uracil phosphoribosyltransferase fusion expression cassette, respectively. Recombination at two Cre recombinase sites on the same chromosome in the same orientation may result in a deletion of the intervening sequence containing the negative selectable markers, for example the herpes simplex virus thymidine kinase expression cassette and cytidine deaminase and uracil phosphoribosyltransferase fusion expression cassette. Drug resistant clones may be grown in sufficient quantities to cryogenically preserve aliquots and to extract DNA from cell clones. Deletions of intervening sequence and the absence of gross chromosomal rearrangements such as duplications may be determined using various methods, for example, by Southern blots, polymerase chain reaction, reverse transcriptase polymerase chain reaction, spectral karyotyping, fluorescence in situ hybridization, multiplex fluorescence in situ hybridization, comparative genomic hybridization. In some variations, cell clones containing a single deletion with no additional chromosomal rearrangements are grown in quantity and cryogenically preserved in aliquots.

In some variations, cell clones containing Cre recombinase sites on the same chromosome are treated with mutagens. Mutagens include but are not limited to: chemical mutagens including but not limited to ethyl methane sulfonate, ethyl nitroso urea, nitrosoguanidine, methyl methanesulfonate, bromouracil, aminopurine, nitrous acid, acridine orange, ethidium bromide, and proflavin; electromagnetic radiation including but not limited to X-ray irradiation, gamma ray irradiation, UV irradiation; and insertional mutagens including but not limited to 5' gene traps, 3' gene traps, transposons, retrotransposons, retroviruses, pro-viral constructs, transgenes, plasmids, and repetitive sequence elements. Since mutagens have different mutational efficiencies and biases, in some variations several mutagens are used separately, in combination, or in varied doses. Individual cells surviving the mutagen treatment may be isolated and grown separately. Methods to individually clone cells include but are not limited to fluorescently activated cell sorting and limited dilution cell culturing. A plurality of cell clones may be grown in sufficient quantities to be cryogenically preserved in aliquots and cultured for further genetic manipulation.

In some variations, DNA containing an expression cassette of a Cre recombinase is introduced into each mutagenized cell clone containing two Cre recombinase sites. Cells clones containing deletions are enriched for by drug selection against negative selectable markers, such as by ganciclovir and 5-fluorocytosine drug selection against the inclusion of the herpes simplex virus thymidine kinase expression cassette and cytidine deaminase and uracil phosphoribosyltransferase fusion expression cassette, respectively. In some variations, drug resistant clones are grown in sufficient quantities to cryogenically preserve aliquots, to extract DNA from cell clones, and to determine phenotypic variant clones. In some variations, deletions of intervening sequence and the absence of gross chromosomal rearrangements such as duplications are determined by means including but not limited to Southern blots, polymerase chain reaction, reverse transcriptase polymerase chain reaction, spectral karyotyping, fluorescence in situ hybridization, multiplex fluorescence in situ hybridization, comparative genomic hybridization. Mutagenized cell clones containing a single deletion with no additional chromosomal rearrangements are cryogenically preserved as well as grown in culture to assay phenotypic variation.

In some variations the DNA is introduced into the isogenized cell clones for random integration using any of the methods described herein or otherwise known by those of skill in the art. In some variations the DNA contains, for example, a neomycin resistance gene expression cassette and a herpes simplex virus thymidine kinase expression cassette. In some variations the DNA contains a site-specific recombination site. Variations with random integration of DNA may have the advantage that large numbers of individual clones containing unique integration sites can be isolated easily. Methods to individually clone cells include but are not limited to fluorescently activated cell sorting and limited dilution cell culturing. Individual cell clones containing selectable markers may be grown in sufficient quantities to cryogenically preserve aliquots and to extract both DNA and RNA from cell clones. In some variations the location of the random integration site into the host genome are identified by to splinkerette PCR, inverse PCR, or reverse transcriptase PCR. In some variations, cell clones containing a single integration site of the selectable markers are used for further manipulation.

In some variations DNA is introduced into isogenized cell clones to integrate in a predetermined site in the genome. In a nonlimiting example of targeting the selectable markers or site-specific recombination sites into the genome, homologous recombination may be used. In some variations the DNA vector to be targeted minimally contain a 5' flanking sequence homologous to endogenous genomic sequence, a neomycin resistance gene expression cassette, a herpes simplex virus thymidine kinase expression cassette, and 3' flanking sequence homologous to endogenous genomic sequence. In some variations a DNA vector with additional functional elements contains, in order, a 5' flanking sequence homologous to an endogenous genomic sequence, a neomycin resistance gene expression cassette, a Cre recombination site, a herpes simplex virus thymidine kinase expression cassette, 3' flanking sequence homologous to endogenous genomic sequence, and an optional diptheria toxin or other negative selectable marker expression cassette. The diptheria toxin expression cassette or other negative selectable marker is optional, as it typically enriches homologous recombination events by only five- to ten-fold. In this variation, cell clones introduced with the DNA construct are selected for based on the selectable marker, for example by a neomycin analog such as G418. In a further variation, individual cell clones are isolated by fluorescent activated cell sorting or limited dilution cell culturing. In a further variation a plurality of cell clones are isolated and are grown in sufficient quantities to cryogenically preserve aliquots to extract DNA from cell clones. In a further variation, individual clones are tested for homologous recombination at the locus determined by the flanking homologous sequences within each individual vector. Methods to determine homologous recombination at each locus include but are not limited to Southern blots and polymerase chain reaction. Clones are identified as containing a Cre recombination site integrated into the genome in a predetermined locus.

In some variations, whether using random integration or homologous recombination at a predetermined locus, cell clones containing a neomycin resistance gene expression cassette and a herpes simplex virus thymidine kinase expression cassette are utilized for further genetic manipulation. In some variations, individual cell clones containing integrated DNA constructs are treated with deletion causing mutagens. Deletion causing mutagens include but are not limited to: electromagnetic radiation including but not limited to X-ray irradiation, UV irradiation, gamma irradiation; chemical mutagens including but not limited to formaldehyde, diepoxyoctane, ethyl nitroso urea, ethyl methane sulfonate, and UV-activated trimethylpsoralen. Individual cell clones can be selected for deletions at the point of integration of the DNA construct. In this non-limiting example ganciclovir drug selection against the inclusion of the herpes simplex virus thymidine kinase expression cassette will select for cell clones containing a deletion at the point of integration. In some variations, drug resistant clones are grown in sufficient quantities to cryogenically preserve aliquots, to extract DNA from cell clones, and to determine phenotypic variant clones.

In some variations, genomic comparison between the parental cell clones and the daughter phenotypically variant cell clones may be used to determine the location of the induced deletion. Genomic comparison means include but are not limited to comparative genomic hybridization, fluorescence in situ hybridization, multiplex fluorescence in situ hybridization, spectral karyotyping, Giemsa stain karyotyping, Southern blot, and gene and genomic array hybridization.

In some variations, whether using random integration or homologous recombination at a predetermined locus, cell clones containing a single Cre recombination site are utilized for further genetic manipulation. In some variations, DNA containing a second Cre recombination site is introduced into cell clones containing an integrated Cre recombination site. In some variations, the DNA vector to be targeted minimally contains a 5' flanking sequence homologous to endogenous genomic sequence, a Cre recombination site, and 3' flanking sequence homologous to endogenous genomic sequence. In some variations a nucleic acid vector with additional functional elements contains one or more of a 5' flanking sequence homologous to endogenous genomic sequence, a negative selectable marker such as a cytidine deaminase and uracil phosphoribosyltransferase fusion expression cassette, a hygromycin resistance gene expression cassette, a Cre recombination site, a visible marker such as a green fluorescent protein expression cassette, a 3' flanking sequence homologous to endogenous genomic sequence, and an optional diptheria toxin expression cassette. The diptheria toxin expression cassette or a comparable negative selectable marker is optional, as it typically enriches homologous recombination events by only five fold. In such a variation, cell clones introduced with the nucleic acid construct may be selected for based on the positive selectable marker, such as a hygromycin-containing medium. In some variations, individual cell clones are isolated by fluorescent activated cell sorting or limited dilution cell culturing. In some variations a plurality of cell clones are isolated and are grown in sufficient quantities to cryogenically preserve aliquots to extract DNA from cell clones. In some variations individual clones are tested for homologous recombination at the locus determined by the flanking homologous sequences within each individual vector. Methods to determine homologous recombination at each locus include but are not limited to Southern blots and polymerase chain reaction. In some variations clones are identified as containing a second Cre recombination site integrated into the genome in a known or predetermined locus.

For diploid cells, the second Cre recombination site will have a 50% probability of integrating into the same chromosome containing the first Cre recombination site, and a 50% probability of integrating into the homologous chromosome. Recombination at two Cre recombination sites on the same chromosome in the same orientation may result in a deletion of the intervening sequence. Recombination at two Cre recombination sites on the same chromosome in the opposite orientation may result in an inversion of the intervening sequence. Recombination at two Cre recombination sites on homologous chromosomes may result in a reciprocal translocation.

In some variations, DNA containing an expression cassette of Cre recombinase is introduced into cell clones containing two Cre recombination sites integrated into the genome at known loci. Cells clones containing deletions are enriched for by drug selection against negative selectable markers, for example by ganciclovir and 5-fluorocytosine drug selection against the inclusion of the herpes simplex virus thymidine kinase expression cassette and cytidine deaminase and uracil phosphoribosyltransferase fusion expression cassette, respectively. Recombination at two Cre recombination sites on the same chromosome in the same orientation may result in a deletion of the intervening sequence containing the negative selectable markers, for example the herpes simplex virus thymidine kinase expression cassette and cytidine deaminase and uracil phosphoribosyltransferase fusion expression cassette. Drug resistant clones may be grown in sufficient quantities to cryogenically preserve aliquots and to extract DNA from cell clones. Deletions of intervening sequence and the absence of gross chromosomal rearrangements such as duplications may be determined using various methods, for example, by Southern blots, polymerase chain reaction, reverse transcriptase polymerase chain reaction, spectral karyotyping, fluorescence in situ hybridization, multiplex fluorescence in situ hybridization, comparative genomic hybridization. In some variations, cell clones containing a single deletion with no additional chromosomal rearrangements are grown in quantity and cryogenically preserved in aliquots.

In some variations, cells contain deletions induced by deletion mutagens, including but not limited to deletion by electromagnetic radiation. In some variations, cells contain deletions induced by site-specific recombinase. In some variations, cells containing deletions are introduced with DNA containing a cytidine deaminase and uracil phosphoribosyltransferase fusion expression cassette, a neomycin resistance gene expression cassette, a Cre recombination site, and a herpes simplex virus thymidine kinase expression cassette. In some variations the integration of the DNA is random. In some variations the integration of the DNA is in a predetermined site within the deletion boundary. In a nonlimiting example of targeting the selectable markers into the genome, homologous recombination may be used, and will include functional elements in the DNA as described herein. Methods to determine homologous recombination at each locus include but are not limited to Southern blots and polymerase chain reaction. Clones are identified as containing the DNA cassette integrated into the genome in a predetermined locus.

In some variations, individual cell clones containing integrated DNA constructs are treated with deletion causing mutagens described herein, including but not limited to electromagnetic radiation such as X-ray irradiation, UV irradiation, and gamma irradiation. Individual cell clones can be selected for deletions at the point of integration of the DNA construct. In this non-limiting example ganciclovir and 5-fluorocytosine drug selection against the inclusion of the herpes simplex virus thymidine kinase expression cassette and cytidine deaminase and uracil phosphoribosyltransferase fusion expression cassette, respectively will select for cell clones containing a deletion at the point of integration. In some variations, drug resistant clones are grown in sufficient quantities to cryogenically preserve aliquots, to extract DNA from cell clones, and to determine phenotypic variant clones.

In some variations, mutagenized cell clones, including but not limited to cancer cell clones or lung cell clones, containing deletions are tested for cytotoxic sensitivity to one or more chemotherapeutic agents including but not limited to cisplatin, carboplatin, paclitaxel, docetaxel, topotecan, irinotecan, vinorelbine, gemcitabine, vincristine, doxorubicin, etoposide, cylophosphamide, mustargen-nitrogen mustard, melphalan, chlorambucil, carmustine, thiotepa, busulfan, vinblastine, actinomycin D, daunorubicin, bleomycin, idarubicin, mitoxantrone, prednisone, triamcinolone, methotrexate, 6-thioguanine, 5-fluorouracil, dacarbazine, cytosine arabinoside, L-asparaginase, hydroxurea, procarbazine. In some variations, cell clones that are more sensitive to single chemotherapeutic agents or combination treatments contain mutations in genes that represent potential drug targets in combination with standard chemotherapeutic agents. In some variations, cell clones that are less sensitive to single chemotherapeutic agents or combination treatments contain mutations in genes that represent potential diagnostic markers for disease treatment or progression.

Growth of in vitro colonies of human lung cells in soft agar is an assay that models anchorage independent growth of human tumors in vivo. In some variations, mutagenized lung cancer cell clones containing deletions are tested for their ability to grow colonies in soft agar. In some variations, cell clones that are less capable of colony formation in soft agar may contain mutations in genes that represent potential drug targets for the growth of human tumors. In some variations, cell clones that are more capable of colony formation in soft agar may contain mutations in genes that represent potential diagnostic markers for disease treatment or progression.

It is hypothesized that tumors need to recruit neovasculature in order to sustain growth. In some variations, mutagenized lung cancer cell clones containing deletions are placed on the chorioallantoic membrane of developing chickens. In one such variation, vessels are visualized around the cell graft and quantified. In some variations, mutagenized lung cancer cell clones containing deletions are tested for their ability to recruit neovasculature when implanted into the corneal stroma adjacent to the temporal limbus in an experimental animal. Experimental animals include but are not limited to mice, rats, guinea pigs, rabbits, cats, dogs, pigs. In some variations, the growth of blood vessels in the implanted region of the eye is visualized directly. In some variations, cell clones that are less capable of recruiting neovasculature may contain mutations in genes that represent potential drug targets for the inhibition of neovascularization of tumors. In some variations, cell clones that are more capable of recruiting neovasculature may contain mutations in genes that represent potential diagnostic markers for disease treatment or progression.

In some variations, mutagenized lung cancer cell clones containing deletions are xenografted into experimental animals. Experimental animals include but are not limited to immune competent or immune compromised mice, rats, guinea pigs, rabbits, cats, dogs, or pigs. In some variations, immune compromised experimental animals are informative experimental hosts where host immune surveillance leads to tumor rejection and heterogeneity in results when implanted cells are not syngeneic with the host. Immune compromised animals include but are not limited to animals with severe combined immune deficiency, surgically or genetically athymic animals, animals with thymic rudiments, animals treated with sub-lethal doses of electromagnetic radiation causing temporary immune deficit, and animals lacking adaptive immune cells or responses. In some variations, xenografts are placed in one or more of ectopic or orthotopic positions. In other variations, intradermal injections or subcutaneous injections of lung cells may allow for the measurement of tumor size and spontaneous metastasis. In some variations, intravenous injection may allow for observation of metastasis.

Methods of orthotopic xenografts include but are not limited to orotracheal intubation co-administration of lung cancer cells with elastase or ethylenediaminetetraacetic acid, use of orotracheal cannulas to intratracheally instill lung cancer cells, endobronchial implantation of lung cancer cells or tissue. In some variations, orthotopic xenografts may allow for the observation of tumor growth, recruitment of neovasculature, and metastases.

In some variations, lung cells expressing visible markers are observed to detect tumor growth, density, and metastases. Methods of observation include but are not limited to positron emission tomography and fluorescent microscopy. In some variations, tumor xenografts are allowed to grow in experimental animals for a prescribed period of time that depends on the tumor cell growth rate. In some variations, tumor growth is monitored while the experimental animal is still alive. In some variations, the animals are sacrificed, and the original tumors and metastases to organs are surgically removed, measured and examined pathologically. In some variations, tumor sites are observed, measured, quantified and scored for the recruitment of neovasculature and penetration of the basement membrane.

In some variations, cells clones that demonstrate one or more of a lesser tumor growth, recruitment of neovasculature, basement membrane penetration, and metastasis to ectopic sites, contain mutations in genes that represent potential drug targets for the inhibition of tumor growth and behavior. In some variations, cells clones that demonstrate one or more of greater tumor growth, recruitment of neovasculature, basement membrane penetration, and metastasis to ectopic sites, may contain mutations in genes that represent potential diagnostic markers for disease treatment or progression.

In some variations, mutagenized lung cancer cell clones containing deletions are compared with the parental isogenized lung cancer cell line. In this way, genetically manipulated cell clones may be assayed for phenotypic variation as enhancement or suppression of tumor growth or other behavior.

In some variations, the identification of mutations in genes in mutagenized lung cancer lines containing a deletion are in comparison to gene sequences in the parental isogenized lung cancer cell line. Methods of gene sequence comparison include but are not limited to hybridization of nucleic acid sequences between mutagenized phenotypically variant lung cancer lines and the parental isogenized lung cancer cell line, and sequencing DNA of mutagenized phenotypically variant lung cancer lines and the parental isogenized lung cancer cell line in the region bounded by site-specific recombination sites.

### Libraries of Cultured Cells

Unless the context clearly indicates otherwise, it is intended that any of the cells prepared or identified by the methods described herein may be collected into one or more cell libraries.

Described herein are libraries of cells as described herein comprising a nucleic acid sequence comprising one or more site-specific recombinase sites. Described herein are libraries of cells having one or more deletions generated by site-specific recombination or by mutagens including but not limited to electromagnetic radiation. Described herein are libraries of cells comprising deletions generated by site-specific recombination or by mutagens including but not limited to electromagnetic radiation, and one or more additional mutations caused by an alternative mutational method described herein.

In some variations, the cellular libraries are used for *in vivo* or *in vitro* phenotyping and gene mapping. In some variations, indexed cell clones having genetically related clones (including but not limited to clones with no genetic alterations, clones which have targeted chromosomes but no deletion, clones with a deletion only, clones with mutations but no deletion, clones with mutations and a deletion) are used for phenotyping and gene mapping. The individual cell clones can be separated and clonally expanded.

In some variations, the extent of mutagenesis covers the entire set of genes in a particular cell or organism within a cellular library. In some variations, by using overlapping sets of deletion points in individual clones, the collective cellular library can contain deletions in about 5-10%, about 10-20%, about 20-30%, about 30-40%, about 40-50%, about 50-60%, about 60-70%, about 70-80%; about 80-90%, about 90-100% of the genome.

In some variations, each cell from a randomly mutagenized population of cells has one or more distinct heterozygous mutations. By combining cell clones that have been randomly mutagenized within the cellular library, mutations on all alleles of the entire set of genes can be collectively represented within a cellular library. Alternatively, by randomly mutagenizing a library containing a population of cells, mutations on all alleles of the entire set of genes can be collectively mutated within the cellular library. The cellular libraries can be indexed to contain genetically related clones. For example, a clonally isogenized cell clone can represent an arbitrarily chosen reference state (for example a cancerous cell, a cell deemed phenotypically mutant compared to analogous cells, cells containing one or more mutations, cells expressing one or more transgenes, cells containing a reporter construct, wild type cells, etc.). One can genetically modify the reference clone to target a chromosome, (for example, with site-specific recombination sites, or positive and negative selectable markers), and unless the targeting disrupts a gene or is phenotypically variant, the resulting clones can be considered "targeted reference." The "targeted reference" clones can be induced to delete a portion of the chromosome, for example, by introducing a site-specific recombinase, or treating with electromagnetic radiation and the resulting clones can be considered "heterozygous by deletion." In some variations "heterozygous by deletion" clones are targeted with site-specific recombination sites and/or positive and negative selectable markers and can be considered "heterozygous by deletion targeted" clones. The "heterozygous by deletion targeted" clones can be induced to delete a portion of the chromosome, for example, by introducing a site-specific recombinase, or treating with electromagnetic radiation and the resulting clones can be considered "homozygous by deletion." In some variations, "targeted reference" clones are treated with mutagens or inhibitors of DNA repair, resulting in one or more mutations and the resulting clones can be considered "heterozygous by mutagen." The "heterozygous by mutagen" clones can be induced to delete a portion of the chromosome (by introducing a site-specific recombinase, or treating with electromagnetic radiation) and the resulting clones can be considered "homozygous." Alternatively, the "heterozygous by.deletion" clones can be treated with mutagens or inhibitors of DNA repair, resulting in one or more mutations and the resulting clones can also be considered "homozygous." The "wild type," "targeted reference," "heterozygous by deletion," "heterozygous by mutagen," "homozygous by deletion," and ""homozygous" cell clones can be indexed in separate libraries.

The number of individual clones in indexed cellular libraries can vary. As few as one clone would suffice for phenotypic or genotypic comparison to other clones. Maintaining several identical copies of a "wild type" clone in a cellular library may provide a redundant resource. In some variations, the number of "targeted reference," "heterozygous by deletion," "heterozygous by mutagen," "homozygous by deletion," and "homozygous" cell clones in a cellular library varies depending on the genomic size and number of genes in each target cell. In some variations, the number of individual cell clones in a cellular library comprises about at least 5, about at least 10, about at least 20, about at least 30, about at least 40, about at least 50, about at least 100, about at least 200, about at least 300, about at least 400, about at least 500, about at least 600, about at least 700, about at least 800, about at least 900, about at least 1,000, about at least 5,000, about at least 10,000, about at least 20,000, about at least 50,000, about at least 100,000, about at least 200,000, or about at least 2,000,000.

In some variations, the number of individual cell clones in a library is between about 1-10, about 10-20, about 20-30, about 30-40, about 40-50, about 50-100, about 100-200, about 200-300, about 300-400, about 400-500, about 500-1,000, about 1,000-5,000, about 5,000-10,000, about 10,000-20,000, about 20,000-50,000, about 50,000-100,000, about 100,000-200,000, about 200,000-2,000,000.

In some variations, cellular libraries collectively contain homozygous mutations in the entire set of genes within the target cell. This enables one to screen for or sequence genes to ensure that every gene is mutated. In some variations, there is saturation mutagenesis of each target cell. Saturation mutagenesis is a genetics term that refers to a statistical situation where there is a high probability (70-99%) that collectively, each gene in the genome is mutated as measured by a standard Poisson distribution. Described herein are cells and cell clones in cellular libraries comprising 10-90%, 20-50%, 50-70%, 70-90%, 10-20%, 20-30%, 30-40%, 40-50%, 50-60%, 60-70%, 70-80%, 80-90%, 90-95%, 90-100% of the genome.

In some variations, separate cellular libraries for different species are maintained. In some variations, separate cellular libraries are maintained for different tissue or cell types (e.g. lung cells versus breast cells): In some variations, separate cellular libraries are maintained for the same cell type (for example, with or without a reporter gene, expressing a gene of interest, or lacking of a gene of interest expression).

In some variations, a cell in a cell library is identified as having a genotypic or phenotypic variation, and the genetic lesion is mapped using the methods described herein.

### Methods of Cell Culture-Based Mapping

Individual cell clones containing induced deletions may contain extensive genomic deletions. The deletion boundaries may be mapped and the genomic interval may be sequenced from the genotypic or phenotypic variant clones and parental clones. However, comparative sequencing of large portions of the genome may be cost prohibitive. In some variations, the region of genetic sequence to be comparatively sequenced is narrowed using one or more methods described herein. In some variations, the region of genetic sequence to be comparatively sequenced is narrowed using one or more methods described herein, until the region to be comparatively sequenced is sufficiently small to render the sequencing cost-effective.

In some variations, cell clones that show phenotypic variation have a recombinase-induced deletion on one chromosome and a mutation (*) on a homologous chromosome (FIG. 2-D). The dependence of the phenotypic variation on the deletion can be demonstrated by testing the cells that have a mutation(*) on one chromosome, but no deletion on the homologous chromosome (FIG. 2-C).

In some variations, cells have a deletion caused by exposing cells comprising two site-specific recombination sites on a chromosome to a site-specific recombinase. In this variation, deletion-bearing cells also comprise one or more additional genetic lesions caused by a separate mutagenesis step, including but not limited to exposure to a chemical mutagen. Cells showing phenotypic variation dependent on the deletion (FIG. 3-A) can be targeted with another site-specific recombination site (10) in the correct orientation between the two previous sites (FIG. 3-B). Introduction of a site-specific recombinase can produce three different deletions as illustrated in FIGS. 3-C, 3-D, 3-E. Cells in FIG. 3-C have a mutation (*) on one chromosome, but the other allele on the homologous chromosome is intact and wild type, so the cells will show no phenotypic variation. Cells in FIGS. 3-D, 3-E have a mutation (*) on one chromosome and the homologous chromosome is deleted at that locus, so these cells will show phenotypic differences. The process illustrated in FIG. 3 can be reiterated to narrow the region containing the mutation, to a point where it is cost effective to comparatively sequence the genomic region.

In order to facilitate the creation of deletions, one or more additional features can be targeted in the chromosome. FIG. 4 depicts a detailed exemplary variation manner of using the methods described herein.

As one example, one or more positive selectable markers (12) may be used to select for the integration of the targeting construct into the genome. Positive selectable markers include but are not limited to the neomycin resistance gene, the β-galactosidase and neomycin resistance gene fusion, the hygromycin resistance gene, puromycin resistance gene, blasticitidin resistance gene, bleomycin resistance gene, guanine phosphoribosyltransferase, hypoxanthine phosphoribosyltransferase, histidinol dehydrogenase, or genes that confer a metabolic or competitive advantage without relying on the treatment of an external agent.

Site-specific recombination sites (10) may be used to create large deletions in the genome; site-specific recombination sites include, but are not limited to, loxP sites, loxP sites with variable spacer regions, FRT sites, attB sites, attP sites, attL sites, or attR sites.

One or more negative selectable markers (16) can be selected against; this allows one to select for the deletion of the chromosome portion containing the marker after treating cells with a site-specific recombinase (FIG. 4-B). Negative selectable markers include, but are not limited to, herpes simplex virus thymidine kinase, hypoxanthine phosphoribosyltransferase, guanine phosphoribosyltransferase, hypoxanthine-guanine phosphoribosyltransferase, adenine phosphoribosyltransferase, cytidine deaminase, uracil phosphoribosyltransferase.

Another feature is the use of one or more reporter genes such as visible markers (18); this class includes, but is not limited to, fluorescent proteins, peptides that can bind to a fluorophore, enzymes that can convert colorimetric or fluorescent substrates, enzymes that can convert substrates into colorimetric or fluorescent products, and peptide or protein targets of antibodies.

One non-limiting example of an ordering of markers and sites on a targeted chromosome is indicated in FIG. 4-A, although the addition, or subtraction of several features, as well as many permutations in the ordering of these features, can offer similar results. Reporter genes such as visible markers (18) lie external to the region of the site-specific recombination sites (10). These visible markers can allow one to identify live cultured cells, segregate or sort individual cell clones, estimate the rate of mutagenesis from mutagen treatment, identify the presence and number of cells when placed in any experimental *in vitro* or *in vivo* model. Internal to the site-specific recombination sites (10) are positive selectable markers (12) that allow for drug or metabolic selection upon integration of each targeting vector. More internal is a negative selectable marker (16) and/or another visible marker (18). After introducing a site-specific recombinase, cells containing deletions can be selected with drug treatment, because only the surviving cell clones will have deleted the intervening chromosome portion containing the negative selectable marker (FIG. 4-B). Alternatively, visible markers (18) can be used to screen for the small number of cells that delete the chromosome portion, and the cell clones can be separated based on the absence of the visible marker. FIG. 4 depicts one method of creating chromosomal deletions.

FIG. 5 shows a further variation of a chromosome targeted with a third compatible site-specific recombination site (10) in the correct orientation. The third targeted portion has similar features. A positive selectable marker (12) allows for drug or metabolic selection upon integration of each targeting vector. Flanking negative selectable markers (16) or visible markers (18) allow for selection of deletions by drug treatment or identification of deletions by their absence, following treatment with a site-specific recombinase. The addition to the chromosome of a third internal site-specific recombination site (10), allows for three possible deletion events when a site-specific recombinase is introduced. In FIG. 5-B the left flank is deleted but the right flank is intact. In FIG. 5-C the right flank is deleted, but the left flank is intact. In FIG. 5-D the deletion removes the entire region between the most extremely located site-specific recombination sites (10). The smaller deletions are favored, because site-specific recombinases are more efficient when the site-specific recombination sites (10) are closer together. Both sets of smaller deletions can be used to narrow the region of the homologous chromosome that contains a lesion causing phenotypic variation. The use of drugs to select for the inclusion of positive selectable markers (12), and for the deletion of negative selectable markers (16), can enrich for specific classes of deletions in cell clones.

Variations on the functional elements in targeted chromosomes will be clear to those skilled in the art based on the teachings herein. These variations are also included within the scope of the methods described herein.

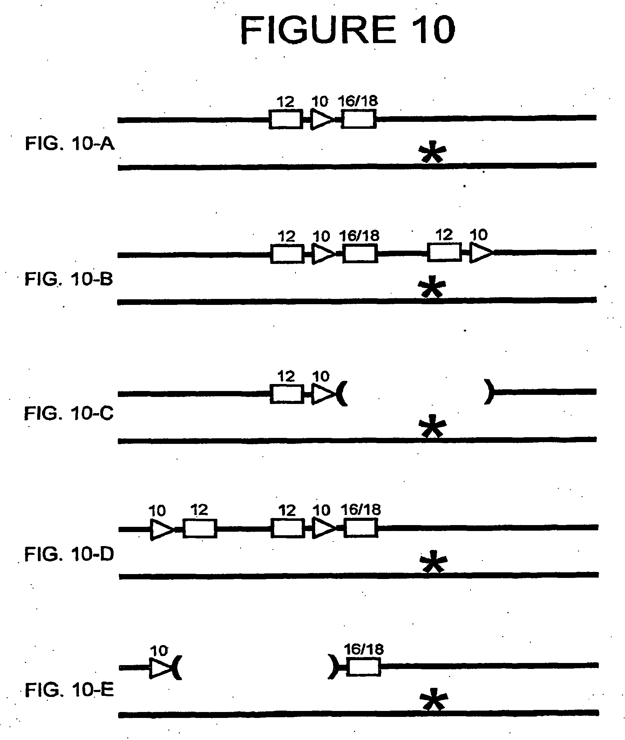

In some variations, methods of mapping a genetic lesion responsible for a genotypic or phenotypic variation are as follows. Individual cell clones containing non-recombinase induced deletions and mutations (FIG. 9-D) which demonstrate genotypic or phenotypic variation are derived from parental cell clones that contain the mutations without the deletion (FIGS. 9-C, 10-A). The parental cell clones (FIG. 10-A) may be further manipulated by the introduction of DNA minimally containing a site-specific recombination site (10). The optional inclusion of a positive selectable marker (12) or a negative selectable marker (16) in the DNA construct may aid in the selection of its integration. In some variations the DNA integrates through homologous recombination and the DNA is targeted to a specific locus. In some variations the DNA integrates randomly into the genome.

In some variations unique sequence tags may be added in the inserted functional elements in the targeted chromosomes. This may allow clones to be combined and screened for phenotypic variance. Subsequently the presence, absence, or abundance of clones may be detected by methods including, but not limited to, hybridization, microarray and PCR.

Cell clones may be screened for containing an additional site-specific recombination site (10) integrated onto the same chromosome as the first site-specific recombination site (10) (FIGS. 10-B, 10-D). Introduction of a site-specific recombinase into these cell clones (FIGS. 10-B, 10-D), may result in deletion of sequence between the site-specific recombination sites (FIGS. 10-C, 10-E). The presence of a negative selectable marker (16) or a visible marker (18) in between the site-specific recombination sites (FIG. 10-B) may aid in selection or screening for deletions. Following the introduction of a site-specific recombinase, cells may be selected against the inclusion of a negative selectable marker (16), for example by selection based on sensitivity to a chemical or reagent, or screened for the absence of a visible marker (18). In some variations, cell clones that have a deletion on one chromosome that uncovers a mutation (*) on the homologous chromosome (FIG. 10-C) may show genotypic or phenotypic variation. In some variations, cell clones that have a deletion on a portion of one chromosome that does not uncover a mutation (*) on the homologous chromosome (FIG. 10-E) will not show genotypic or phenotypic variation. The process illustrated in FIG. 10 may be reiterated to narrow the region containing the mutation, to a point where it is cost effective to comparatively sequence the genomic region to determine the genetic sequence that is correlated with the genotypic or phenotypic variation.

In some variations, methods of mapping a genetic lesion responsible for a genotypic or phenotypic variation are as follows. Individual cell clones containing heteroallelic deletions (FIGS. 12-D, 12-E) that demonstrate genotypic or phenotypic variation are derived from parental cell clones that contain a deletion on one chromosome (FIGS. 12-C, 13-A). The parental cell clones (FIG. 13-A) may be further manipulated by the introduction of DNA minimally containing a site-specific recombination site (10). The optional inclusion of a positive selectable marker (12) or a negative selectable marker in the DNA construct may aid in the selection of its integration. In some variations the DNA integrates through homologous recombination and the DNA is targeted to a specific locus. In some variations the DNA integrates randomly into the genome.

Cell clones may be screened for containing an additional site-specific recombination site (10) integrated onto the same chromosome as the first site-specific recombination site (10) (FIGS. 13-B, 13-D). Introduction of a site-specific recombinase into these cell clones (FIGS. 13-B, 13-D) may result in deletion of sequence between the site-specific recombination sites (FIGS. 13-C, 13-E). The presence of a negative selectable marker (16) or a visible marker (18) in between the site-specific recombination sites (FIGS. 13-B, 13-D) may aid in selection or screening for deletions. Following the introduction of a site-specific recombinase, cells may be selected against the inclusion of a negative selectable marker (16), for example by selection based on sensitivity to a chemical or reagent, or screened for the absence of a visible marker (18). In some variations, cell clones that have a heteroallelic deletion combination in a chromosomal segment (FIG. 13-C) may show phenotypic variation, while cell clones that have a heteroallelic deletion combination in a neighboring chromosomal segment (FIG. 13-E) will not show phenotypic variation. The process illustrated in FIG. 13 may be reiterated to narrow the region containing the heteroallelic deletion, to determine the genetic sequence that is correlated with the phenotypic variation.

In some variations, methods of mapping a genetic lesion responsible for a genotypic or phenotypic variation are as follows. Individual cell clones containing heteroallelic deletions are derived from cells containing a deletion and a site-specific recombination site on the corresponding homologous chromosome segment (FIG. 15-A). These cells are introduced with DNA containing a positive selectable marker (12) and a site-specific recombination site (10) (FIGS. 15-B, 15-D). In some variations site-specific recombinase is introduced to cells containing two site-specific recombination sites (10) in the same orientation (FIGS. 15-B, 15-D), resulting in smaller heteroallelic deletions (FIGS. 15-C, 15-E). In some variations, cell clones that have a heteroallelic deletion combination in a chromosomal segment (FIG. 15-C) may show phenotypic variation, while cell clones that have a heteroallelic deletion combination in a neighboring chromosomal segment (FIG. 15-E) will not show phenotypic variation. The process illustrated in FIG. 15 may be reiterated to narrow the region containing the heteroallelic deletion, to determine the genetic sequence that is correlated with the phenotypic variation.

The methods of identifying a genetic sequence correlated with a genotypic or phenotypic variation described herein also include site-specific recombination sites comprising DNA elements including, but not limited to, gene traps, transposons, retrotransposons, retroviruses, pro-viral constructs, transgenes, plasmids, and repetitive sequence elements. These methods can be adapted to gene trap mutagenesis, since the inserted DNA element commonly contains a site-specific recombination site (10), a positive selectable marker (12), a visible marker (18), or a fusion of a positive selectable marker and a visible marker (12:18). Cells containing gene traps may be treated with mutagens or inhibitors of DNA repair to induce mutations at random. Cell clones containing gene traps and randomly induced mutations may be treated with electromagnetic radiation to induce deletions. Deletions at the point of gene trap integration may be drug selected for the deletion of a negative selectable marker (16) in the gene trap, or may be screened for the absence of a visible marker (18) in the gene trap. Cell clones comprising induced deletions and mutations may be screened for genotypic or phenotypic variation in cell culture. Parental cell clones of genotypic or phenotypic variant cell clones may be introduced with a DNA construct containing a site-specific recombination site (10). Cell clones may be screened for containing an additional site-specific recombination site (10) integrated onto the same chromosome as the first site-specific recombination site (10). Introduction of a site-specific recombinase into these cell clones may result in deletion of sequence between the site-specific recombination sites. Deletion containing clones may be screened for the genotypic or phenotypic variation. By reiterating this method, deletions may used to map interval containing the randomly induced mutation responsible for phenotypic variation.

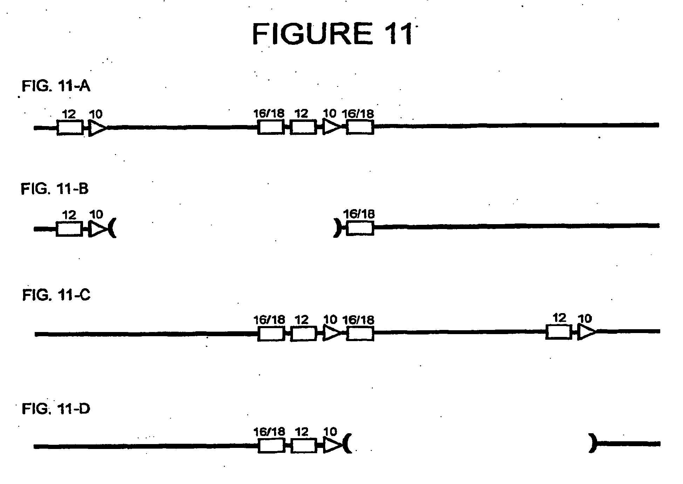

Additional features may be included in the DNA constructs illustrated in FIGS. 9 and 10, including but not limited to those described herein. The initial integrated DNA construct may contain, for example, distinct negative selectable markers (16) and / or visible markers (18) on each side of the site-specific recombination site (10) (FIGS. 11-A, 11-C). DNA constructs that are subsequently integrated in the cell clones may contain distinct negative selectable markers (16) or visible markers (18) on the side between the site-specific recombination sites (10) (FIGS. 11-A, 11-C). The presence of these distinct markers may aid in selecting or screening for subsequent site-specific recombinase induced deletions in the 5' direction (FIG. 11-B) or the 3' direction (FIG. 11-D).

In some variations, homologous recombination is used to introduce the site-specific recombination sites, using a homologous 5' sequence, an internal sequence minimally containing a site-specific recombination site, and a homologous 3' sequence. In some variations, the internal sequence also optionally contains one or more or a positive selectable markers, optionally one or more negative selectable markers, optionally one or more reporter genes, and/or optionally one or more visible markers. In some variations, such additional sequences are on the 3' side of the 3' homologous sequence and aid in targeting efficiency. In some variations, it is possible, and even advisable in cell types with low rates of homologous recombination, to introduce the sequences by two or more events. In some variations, the first event comprises introducing the homologous 5' sequence and the internal sequence containing an incomplete 5' portion of the positive selectable marker. In some variations, the second event comprises introducing one or more of an overlapping yet incomplete 3' portion of the positive selectable marker, the rest of the internal sequences, the homologous 3' sequence, or a negative selectable marker. Introducing the positive selectable marker in two events can help to select for cells that utilize homologous recombination to restore a complete positive selectable marker, which can enrich for correct integration of the sequences into the genome by homologous recombination. Homologous recombination is one method of introducing the site-specific recombination sites into the genome; however, other methods can also be utilized.

In some variations, random integration of a sequence containing a site-specific recombination site is used to introduce one or more site-specific recombination sites.

In some variations, cells are targeted to contain two site-specific recombination sites on one chromosome. While there is no strict limit to the distance between the two sites, some reports indicate that deletions of sequences greater than 3-4 cM can cause gross chromosomal rearrangements including duplications. In some variations, two sites are targeted in pairs in the correct orientation along each chromosome to cover the entire genome in different cell clones. In some variations, cells with two recombination sites on one chromosome are mutagenized with chemical mutagens like ethylmethane sulphonate (EMS) or ethylnitrosourea (ENU), or treated with inhibitors of DNA repair. In some variations, a plurality of mutagenized individual clones is grown in separate cell cultures. In some variations, each clone contains different collections of mutations along the chromosomes. Site-specific recombinase can be introduced to each clone to produce a deletion. In clones where mutations exist which affect genes on the chromosomal segment that is deleted in the homologous chromosome, recessive phenotypes are expected to appear.

Cell clones can then be screened for genotypic or phenotypic variation, and the genetic lesion correlated with the genotypic or phenotypic variation can be mapped.

Genotypic variation includes but is not limited to genetic insertions, deletions, mutations, inversions, polymorphisms, duplications, or epigenetic phenomena.

The term "phenotypic variation," as used herein, includes any qualitative or quantitative difference that can be observed between cell clones. Non-limiting examples of observable phenotypes include: changes in the amount of endogenous cell products as compared to reference cells or wild type; the presence, absence, or difference in cell response or behavior to a stimulus or condition; or altered levels of reporter gene response as compared to a reference cell introduced with a reporter gene. In some variations, phenotypes involve the presence or absence of either constitutive, developmental, or externally induced reporter gene responses. In some variations, phenotypes include the cellular level of mRNA, proteins, lipids, or carbohydrates, including alterations in such levels relative to references or wild type cells; modifications of these molecules, or their biological activities. In some variations, phenotypes involve the developmental potential of a cell, or the specific functions of a specialized cell type. For example, a deletion containing, mutagenized stem cell can be assessed for its ability to differentiate into a pancreatic ß-islet cell, or for its ability to secrete insulin. In some variations, human cells are screened in disease-relevant assays. In some variations, cells are screened for the enhancement or suppression of phenotypes or characteristics associated with a specific disease genotype or phenotype.

### Detailed Discussion of Cell Culture-Based Mapping

In some variations, the amount of nucleic acid sequence to be compared is reduced by mapping the causative genetic lesion to a smaller sequence interval. In some variations, phenotypically variant lung cancer cells contain mutations from mutagenesis and a deletion in the homologous chromosome. In this variation, the cells are derived from a parental cell clone that contains mutations from mutagenesis and a homologous chromosome with at least two Cre recombinase sites, but no deletion via recombinase. In this variation, the parental cell clones are used for further genetic manipulation. In some variations, DNA containing a third Cre recombinase site is introduced into mutagenized cell clones containing two integrated Cre recombinase sites. In this variation, the DNA vector to be targeted minimally contains a 5' - flanking sequence homologous to endogenous genomic sequence, a Cre recombinase site, and 3' flanking sequence homologous to endogenous genomic sequence. In some variations, DNA vectors include additional functional elements, such as one or more of a 5' flanking sequence homologous to endogenous genomic sequence, a negative selectable marker such as a hypoxanthine phosphoribosyltransferase gene expression cassette, a Cre recombinase site, a positive selectable marker such as a puromycin resistance gene expression cassette, 3' flanking sequence homologous to endogenous genomic sequence, and a diptheria toxin gene expression cassette. The diptheria toxin expression cassette or a comparable negative selectable marker is optional, as it typically enriches homologous recombination events by only five fold. In some variations, cell clones introduced with the DNA construct are selected for by selective media such as a puromycin-containing medium. In a further variation, positively selected cells are individually cloned. In a further variation, individual cell clones are isolated by fluorescent activated cell sorting or limited dilution cell culturing, and a plurality of the cell clones isolated and grown in sufficient quantities to cryogenically preserve aliquots from which to extract DNA. In some variations, individual clones are tested for homologous recombination at the locus determined by the flanking homologous sequences within each individual vector. Methods to determine homologous recombination at each locus include but are not limited to Southern blots and polymerase chain reaction. Clones are identified as containing a third Cre recombinase site integrated into the genome in a predetermined locus.

In a diploid cell the third Cre recombinase site will have a 50% probability of integrating into the same chromosome containing the other two Cre recombinase sites, and a 50% probability of integrating into the homologous chromosome. Recombination at two Cre recombinase sites on the same chromosome in the same orientation may result in a deletion of the intervening sequence. Recombination at two Cre recombinase sites on the same chromosome in the opposite orientation may result in an inversion of the intervening sequence. Recombination at two Cre recombinase sites on homologous chromosomes may result in a reciprocal translocation.

In some variations, DNA containing an expression cassette of a Cre recombinase is introduced into cell clones containing three Cre recombinase sites integrated into the genome at known loci. In such a variation, cells clones containing deletions are enriched for by drug selection against the inclusion of negative selectable markers. In some variations, clones that have distinct negative selectable markers internal to the two extreme site-specific recombination sites are selected with one or the other, but not both, corresponding drugs to enrich for specific deletions. In some variations, recombination at the first and second site-specific recombination sites on the same chromosome in the same orientation will result in a deletion of the intervening sequence containing one negative selectable marker, but the sequence between the second and third site-specific recombination sites containing the other negative selectable marker will remain intact. In some variations, recombination at the second and third site-specific recombination sites on the same chromosome in the same orientation will result in a deletion of the intervening sequence containing one negative selectable marker, but the sequence between the first and second site-specific recombination sites containing the other negative selectable marker remains intact. For example, in the specific variation described above, recombination at the first and second Cre recombinase sites on the same chromosome in the same orientation will result in a deletion of the intervening sequence containing a neomycin resistance gene expression cassette, a herpes simplex virus thymidine kinase expression cassette, and a hypoxanthine phosphoribosyltransferase gene expression cassette, while intact sequences include a puromycin resistance gene expression cassette, a cytidine deaminase and uracil phosphoribosyltransferase fusion expression cassette, and a hygromycin resistance gene expression cassette; cell clones containing this deletion are G418 sensitive, ganciclovir resistant, hypoxanthine sensitive, 6-thioguanine resistant, puromycin resistant, 5-flurocytosine sensitive, and hygromycin resistant. Recombination at the second and third Cre recombinase sites on the same chromosome in the same orientation results in a deletion of the intervening sequence containing a puromycin resistance gene expression cassette, a cytidine deaminase and uracil phosphoribosyltransferase fusion expression cassette, while intact sequences include a neomycin resistance gene expression cassette, a herpes simplex virus thymidine kinase expression cassette, and a hypoxanthine phosphoribosyltransferase gene expression cassette; cell clones containing this deletion are G418 resistant, ganciclovir sensitive, hypoxanthine resistant, 6-thioguanine sensitive, puromycin sensitive, 5-flurocytosine resistant, and hygromycin sensitive.

Therefore specific deletions are enriched by selecting with a drug specific to each distinct selectable marker. The smaller deletions will likely be favored because site-specific recombinase is shown to be dependent on the distance between Cre recombinase sites. In some variations, cell clones containing smaller deletions are determined by duplicate plating. In this variation, clones are resistant to drug selection where negative selectable marker sequences are deleted and positive selectable marker sequences remain intact; the same clones may be tested in a duplicate culture for sensitivity to drug selection where positive selectable marker sequences are deleted and negative selectable marker sequences remain intact. Drug resistant clones may be grown in sufficient quantities to cryogenically preserve aliquots and to extract DNA from cell clones. In some variations, deletions of intervening sequence and the absence of gross chromosomal rearrangements such as duplications are determined by means including but not limited to Southern blots, polymerase chain reaction, reverse transcriptase polymerase chain reaction, spectral karyotyping, fluorescence in situ hybridization, multiplex fluorescence in situ hybridization, comparative genomic hybridization. In some variations, cell clones containing a single deletion with no additional chromosomal rearrangements are grown in sufficient quantities to test for phenotypic variation and cryogenically preserved in aliquots.

In some variations, cell clones containing a mutation on one chromosome and a deletion in the corresponding portion of the homologous chromosome are phenotypically variant. In this variation, cell clones containing a mutation on one chromosome, but having an intact corresponding portion of the homologous chromosome with a deletion in a more remote portion, will not be phenotypically variant from the isogenized parental cell clone. In this manner the sequence interval containing the causative genetic lesion are narrowed. In some variations this process are performed in parallel or repeated sequentially to narrow the interval to a convenient size to compare nucleic acid sequences between the mutagenized variant cell clone and the original isogenized cell clone.

In some variations cells may be genetically manipulated by alternate means. The following non-limiting example discusses an alternate means of inducing deletions and an alternate strategy of mapping mutations.

In some variations, lung cancer cells are individually cloned to isogenize the starting population of cells as described above.

In some variations, DNA containing a first site-specific recombination site such as any described herein are introduced into isogenized cell clones using any of the methods described herein or otherwise known by those of skill in the art. Again, the non-limiting example of a Cre recombinase site is used in the following discussion; however, any site-specific recombination site may be used in the methods described herein.

In some variations the DNA is introduced into the isogenized cell clones for integration in a random manner. Methods to individually clone cells include but are not limited to fluorescence activated cell sorting and limited dilution cell culturing. Individual cell clones containing Cre recombination sites may be grown in sufficient quantities to cryogenically preserve aliquots and to extract both DNA and RNA from cell clones. In some variations a gene trap construct may be used to contain a splice acceptor site, or spice donor site within the DNA construct containing the Cre recombinase site. In a further variation the location of the random integration site into the host genome are identified by splinkerette PCR, inverse PCR, or reverse transcriptase PCR. In some variations, cell clones containing a single integration site of a Cre recombination site are used for further manipulation.

In some variations the copy number of integrated plasmids or transgenes is reduced, including reduction to a single copy. In some variations, the copy number can be reduced by introduction of the Cre recombinase. In some variations integrations are not manipulated by Cre recombinase or by any other mechanism to reduce copy numbers.

In the case of random integration of introduced DNA constructs, a non-limiting example of a DNA construct minimally contains a puromycin resistance gene expression cassette, a Cre recombinase site, and a herpes simplex virus thymidine kinase expression cassette. In some variations a DNA vector with additional functional elements may contain, in order, a cytidine deaminase and uracil phosphoribosyltransferase fusion expression cassette, a puromycin resistance gene expression cassette, a Cre recombinase site, and a herpes simplex virus thymidine kinase expression cassette. In such a variation, cell clones introduced with the nucleic acid construct may be selected for directional deletions used to map mutations described herein. In this non-limiting example the cell clones introduced with the DNA construct may be selected for based on the positive selectable marker, such as in a puromycin-containing medium. In some variations, individual cell clones are isolated by fluorescence activated cell sorting or limited dilution cell culturing. In some variations a plurality of cell clones are isolated and are grown in sufficient quantities to cryogenically preserve aliquots to extract DNA from cell clones and for further growth.

In some variations DNA is introduced into isogenized cell clones to integrate a Cre recombinase site in a predetermined site in the genome, using one or more of methods known to those of skill in the art or described herein. In a non-limiting example of targeting a site-specific recombinase site into the genome, homologous recombination may be used. In some variations the DNA vector to be targeted minimally contains a 5' flanking sequence homologous to an endogenous genomic sequence, a puromycin resistance gene expression cassette, a Cre recombinase site, a herpes simplex virus thymidine kinase expression cassette, and 3' flanking sequence homologous to an endogenous genomic sequence. In some variations a DNA vector with additional functional elements may contain, in order, a 5' flanking sequence homologous to endogenous genomic sequence, a cytidine deaminase and uracil phosphoribosyltransferase fusion expression cassette, a puromycin resistance gene expression cassette, a Cre recombinase site, a herpes simplex virus thymidine kinase expression cassette, a 3' flanking sequence homologous to endogenous genomic sequence, and an optional diptheria toxin or other negative selectable marker expression cassette. The diptheria toxin expression cassette or other negative selectable marker is optional, as it typically enriches homologous recombination events by only five- to ten-fold. In this variation, cell clones introduced with the DNA construct are selected for based on the selectable marker, for example by puromycin-containing media. In a further variation, individual cell clones are isolated by fluorescence activated cell sorting or limited dilution cell culturing. In a further variation a plurality of cell clones is isolated and grown in sufficient quantities to cryogenically preserve aliquots to extract DNA from cell clones. In a further variation, individual clones are tested for homologous recombination at the locus determined by the flanking homologous sequences within each individual vector. Methods to determine homologous recombination at each locus include but are not limited to Southern blots and polymerase chain reaction. Clones are identified as containing a Cre recombinase site integrated into the genome in a predetermined locus.

In some variations, individual cell clones containing an integrated DNA construct are treated with mutagens. Mutagens include one or more of those described herein or known by those of skill in the art. Since mutagens have different mutational efficiencies and biases, in some variations several mutagens are used separately, in combination, or in varied doses. Individual cells surviving the mutagen treatment may be isolated and grown separately. Methods to individually clone cells include but are not limited to fluorescence activated cell sorting and limited dilution cell culturing. A plurality of cell clones may be grown in sufficient quantities to be cryogenically preserved in aliquots and cultured for further genetic manipulation. In some variations cells may be treated with mutagens prior to the introduction of a DNA construct containing a positive selectable marker, a site-specific recombination site, and a negative selectable marker.

In some variations, individual cell clones containing integrated DNA constructs and randomly induced mutations are treated with deletion causing mutagens. Deletion causing mutagens include but are not limited to: electromagnetic radiation including but not limited to X-ray irradiation, UV irradiation, gamma irradiation; chemical mutagens including but not limited to formaldehyde, diepoxyoctane, ethyl nitroso urea, ethyl methane sulfonate, and UV-activated trimethylpsoralen. Individual cell clones can be selected for deletions at the point of integration of the DNA construct. In this non-limiting example ganciclovir and 5-fluorocytosine drug selection against the inclusion of the herpes simplex virus thymidine kinase expression cassette and cytidine deaminase and uracil phosphoribosyltransferase fusion expression cassette, respectively will select for cell clones containing a deletion at the point of integration. In some variations, drug resistant clones are grown in sufficient quantities to cryogenically preserve aliquots, to extract DNA from cell clones, and to determine phenotypic variant clones.

In some variations, mutagenized cell clones, including but not limited to cancer cell clones or lung cell clones, containing deletions are tested for cytotoxic sensitivity as described hereinabove.

In some variations, in vitro systems are used to screen for deletions as described herein, including but not limited to such models or systems as soft agar growth. In some variations, mutagenized lung cancer cell clones containing deletions are tested in one or more in vivo models as described herein, including but not limited to tested for their ability to recruit neovasculature when implanted into the corneal stroma adjacent to the temporal limbus in an experimental animal, or into the chorioallantoic membrane of developing chickens.

In some variations, mutagenized lung cancer cell clones containing deletions are xenografted into experimental animals, including but not limited to various immune competent or immune comprosmised experimental animals known by those of skill in the art or described herein. In some variations, xenografts are placed in one or more of ectopic or orthotopic positions. In other variations, intradermal injections or subcutaneous injections of lung cells may allow for the measurement of tumor size and spontaneous metastasis. In some variations, intravenous injection may allow for observation of metastasis. Such systems or models are observed to detect one or more of genotypic or phenotypic variation as described herein.

In some variations, lung cells expressing visible markers are observed to detect tumor growth, density, and metastases using one or more of any methods known by those of skill in the art or described herein.

In some variations, mutagenized lung cancer cell clones containing deletions are compared with the parental isogenized lung cancer cell line. In this way, genetically manipulated cell clones may be assayed for phenotypic variation as enhancement or suppression of tumor growth or other behavior. In some variations, the identification of mutations in genes in mutagenized lung cancer lines containing a deletion are in comparison to gene sequences in the parental isogenized lung cancer cell line, using any one or more of methods known in the art or described herein.

In some variations, the amount of nucleic acid sequence to be compared is reduced by mapping the causative genetic lesion to a smaller sequence interval. In some variations, phenotypic variant lung cancer cells contain mutations from mutagenesis and a deletion in the homologous chromosome. Cells may be tested for phenotypic variation without knowledge of the DNA construct integration site, or the boundaries of the subsequent deletion at this site. In this variation, the cells are derived from a parental cell clone that contains mutations from mutagenesis and a homologous chromosome with an integrated DNA construct containing a Cre recombinase site, but no deletion. In this variation, the parental cell clones are used for further genotypic comparison and genetic manipulation. The site of DNA construct integration may be determined in parental cell clones by means including but not limited to splinkerette PCR, inverse PCR, reverse transcriptase PCR, Southern blot. In some variations, genomic comparison between the parental cell clones and the daughter phenotypically variant cell clones may be determined. Genomic comparison means include but are not limited to comparative genomic hybridization, fluorescence in situ hybridization, multiplex fluorescence in situ hybridization, spectral karyotyping, Giemsa stain karyotyping, Southern blot, and gene and genomic array hybridization.

In some variations, identifying the site of DNA construct integration or the site of the deletion may aid in the subsequent mapping of mutations responsible for phenotypic variation. In this non-limiting example an additional DNA construct containing a Cre recombinase site is introduced into the parental cell clones of phenotypic variant cell clones. In some variations the integration is targeted by homologous recombination. In this variation the DNA vector contains, in order, a 5' flanking sequence homologous to endogenous genomic sequence, a hygromycin resistance gene expression cassette, a Cre recombinase site, a 3' flanking sequence homologous to endogenous genomic sequence, and a diptheria toxin gene expression cassette. The diptheria toxin expression cassette or a comparable negative selectable marker is optional, as it typically enriches homologous recombination events by only five fold. In some variations, cell clones introduced with the DNA construct are selected for by selective media such as a hygromycin-containing medium. In a further variation, positively selected cells are individually cloned. In a further variation, individual cell clones are isolated by fluorescence activated cell sorting or limited dilution cell culturing, and a plurality of the cell clones isolated and grown in sufficient quantities to cryogenically preserve aliquots and from which to extract DNA. In some variations, individual clones are tested for homologous recombination at the locus determined by the flanking homologous sequences within each individual vector. Methods to determine homologous recombination at each locus include but are not limited to Southern blots and polymerase chain reaction. Clones may be identified as containing a second Cre recombinase site integrated into the genome in a predetermined locus.

In diploid cells the second Cre recombinase site will have a 50% probability of integrating into the same chromosome containing the other Cre recombinase site, and a 50% probability of integrating into the homologous chromosome. Recombination at two Cre recombinase sites on the same chromosome in the same orientation may result in a deletion of the intervening sequence. Recombination at two Cre recombinase sites on the same chromosome in the opposite orientation may result in an inversion of the intervening sequence. Recombination at two Cre recombinase sites on homologous chromosomes may result in a reciprocal translocation.

In some variations, DNA containing an expression cassette of a Cre recombinase is introduced into cell clones containing two Cre recombinase sites integrated into the genome in the same orientation at known loci. In such a variation, cells clones containing deletions are enriched for by drug selection against the inclusion of negative selectable markers. In some variations, clones that have distinct negative selectable markers internal to the two extreme site-specific recombination sites are selected with one or the other, but not both, corresponding drugs to enrich for specific deletions. In this non-limiting example the use of 5-fluorocytosine or ganciclovir for selection depends on whether the second Cre recombinase site integrates 5' or 3' respectively in reference to the first Cre recombinase site integration. In some variations the second Cre recombinase site integrates 5' to the original Cre recombinase site integration and the cytidine deaminase and uracil phosphoribosyltransferase fusion expression cassette is flanked by both Cre recombinase sites; in this variation deletions can be selected with 5-fluorocytosine. In some variations the second Cre recombinase site integrates 3' to the original Cre recombinase site integration and the herpes simplex virus thymidine kinase expression cassette is flanked by both Cre recombinase sites; in this variation deletions can be selected with ganciclovir. Drug resistant cell clones may be tested for Cre recombinase induced deletion by means including but not limited to polymerase chain reaction, comparative genomic hybridization, fluorescence in situ hybridization, multiplex fluorescence in situ hybridization, spectral karyotyping, Giemsa stain karyotyping, Southern blot, and gene and genomic array hybridization. Cell clones containing Cre recombinase induced deletions and induced mutations may be grown in sufficient quantities to screen for phenotypic variation and to cryogenically preserve clones in aliquots.

In some variations, cell clones containing a mutation on one chromosome and a deletion in the corresponding portion of the homologous chromosome are phenotypically variant. In this variation, cell clones containing a mutation on one chromosome, but having an intact corresponding portion of the homologous chromosome with a deletion in a more remote portion, will not be phenotypically variant from the isogenized parental cell clone. In this manner the sequence interval containing the causative genetic lesion is narrowed. In some variations this process is performed in parallel or repeated sequentially to narrow the interval to a convenient size to compare nucleic acid sequences between the mutagenized variant cell clone and the original isogenized cell clone.

In some variations, cell clones that show phenotypic variation contain heteroallelic deletions. In some variations, the cells are derived from a parental cell clone that contains a deletion on one chromosome and an integrated DNA cassette containing a site-specific recombination site and a negative selectable marker in the homologous chromosome region. In one nonlimiting example, cells contain a cytidine deaminase and uracil phosphoribosyltransferase fusion expression cassette, a neomycin resistance gene expression cassette, a Cre recombination site, and a herpes simplex virus thymidine kinase expression cassette. DNA containing a positive selectable marker such as a puromycin resistance expression cassette and a Cre recombination site can be introduced into these cells. In some variations the integration of the DNA is random. In some variations the integration of the DNA is in a predetermined site within the deletion boundary. In a nonlimiting example of targeting the selectable markers into the genome, homologous recombination may be used, and will include functional elements in the DNA as described herein. Methods to determine homologous recombination at each locus include but are not limited to Southern blots and polymerase chain reaction. Clones are identified as containing the DNA cassette integrated into the genome in a predetermined locus.

In some variations the second Cre recombination site is integrated 5' to the original Cre recombination site, while in some variations the second Cre recombination site is integrated 3' to the original Cre recombination site.

In some variations, DNA containing an expression cassette of Cre recombinase is introduced into cell clones containing two Cre recombination sites integrated into the genome at known loci. Cells clones containing smaller deletions are enriched for by drug selection against negative selectable markers, for example by 5-fluorocytosine and ganciclovir drug selection against the inclusion of the cytidine deaminase and uracil phosphoribosyltransferase fusion expression cassette and herpes simplex virus thymidine kinase expression cassette, respectively. In this nonlimiting example, recombination at two Cre recombination sites on the same chromosome in the same orientation may result in a small 5' deletion of the intervening sequence containing the cytidine deaminase and uracil phosphoribosyltransferase fusion expression cassette, or a small 3' deletion of the intervening sequence containing the herpes simplex virus thymidine kinase expression cassette. Drug resistant clones may be grown in sufficient quantities to cryogenically preserve aliquots and to extract DNA from cell clones. Deletions of intervening sequence and the absence of gross chromosomal rearrangements such as duplications may be determined using various methods described herein. In some variations, mutagenized cell clones containing a heteroallelic deletion with no additional chromosomal rearrangements are cryogenically preserved as well as grown in culture to assay phenotypic variation. In this nonlimiting example cells containing the smaller 5' deletion may not show phenotypic variation, whereas cells containing the smaller 3' deletion may show phenotypic variation. The process of integrating another Cre recombination site and inducing a deletion can be reiterative, narrowing the region containing the deleted genetic element responsible for the phenotypic variation.

## Claims

1. A method of determining the location or identity of a genetic sequence correlated with a recessive genotypic or phenotypic variation in cultured cells, the genetic sequence located between two inserted site-specific recombination sites, comprising:
(a) obtaining cells comprising two inserted site-specific recombination sites on a chromosome, further comprising a mutation that is correlated with a genotypic or phenotypic variation, wherein the mutation is on a homologous chromosome and bounded by the location of the site-specific recombination sites on the homolog;
(b) inserting a third site-specific recombination site at a known location between the two site-specific recombination sites in a population of the cells of (a);
(c) exposing the cells of (a) to a site-specific recombinase to generate cells comprising at least one deletion;
(d) exposing the cells of (b) to a site-specific recombinase to generate cells comprising at least one deletion;
(e) screening in cultured cells or ex vivo the deletion-containing cells of (c) relative to the deletion-containing cells of (d) for the presence or absence of the phenotypic or genotypic variation;
(f) repeating steps (a)-(c), wherein the third site-specific recombination site is altered relative to that of the first iteration of steps (a)-(c);
(g) determining the location or identity of a genetic sequence that is correlated with the recessive genotypic or phenotypic variation in the cultured cells or ex vivo.

2. The method of claim 1, wherein the third site-specific recombination site is altered relative to that of the first iteration of steps (a)-(c) to narrow the size of the deletion that is correlated with the genotypic or phenotypic variation in the cells, wherein the mutation is located at a site on the homologous chromosome corresponding to the deletion.

3. A method of identifying a genotypic or phenotypic variation, comprising:
screening cells cultured or ex vivo for genotypic or phenotypic variation between:
(a) greater than haploid cells with a recombinase-induced deletion between two inserted site-specific recombination sites in the chromosomal DNA of cultured cells, wherein the DNA of the cells comprises a further mutation in a chromosome homologous with at least a portion of the recombinase-induced deleted sequence, wherein the further mutation was introduced with a method other than recombinase-induced deletion; and
(b) cells selected from the group consisting of:
(i) progenitor cells of the cells of (a) without the recombinase-induced deletion in the chromosomal DNA or the further mutation;
(ii) progenitor cells of the cells of (a) having the recombinase-induced deletion in the chromosomal DNA but absent the further mutation;
(iii) progenitor cells of the cells of (a) having the introduced further mutation but absent the recombinase-induced deletion in the chromosomal DNA, and
(iv) cells other than progenitor cells of the cells of (a)
wherein the location or identity of the genotypic or phenotypic variation is localized to a region of a genetic sequence correlated with the genotypic or phenotypic variation using the method of claim 2.

4. The method of claim 3, wherein the further mutation is via chemical mutagenesis, or wherein the further mutagenesis is by one or more of electromagnetic radiation, treatment with an inhibitor of DNA repair, or insertional mutagenesis.

5. The method of claim 4,
wherein the further method of mutation of the cultured cells of (a) is performed either prior to, concurrently with, or subsequent to the recombinase-induced deletion; or
wherein the site-specific recombinase is Cre recombinase; or
wherein the cells prior to mutagenesis closely adhere to a diploid karyotype; or wherein the phenotypic variation is a member selected from the group consisting of an altered ability to grow colonies in semi-solid medium, altered resistance to chemotherapeutic agents, altered induction of neo-angiogenesis, and an altered ability to metastasize.

6. A method of determining the location or identity of a genetic sequence correlated with a recessive genotypic or phenotypic variation in cultured cells or ex vivo comprising:
(a) obtaining cells comprising an inserted first site-specific recombination site, further comprising a mutation that is correlated with a genotypic or phenotypic variation, wherein the mutation is on a homologous chromosome;
(b) introducing a second site-specific recombination site onto the chromosome comprising the first site-specific recombination site in a population of cells of (a) wherein the location of the mutation on the homologous chromosome is bounded by the location of the site-specific recombination sites on the homo log;
(c) exposing the cells of (b) to a site-specific recombinase to generate cells comprising at least one deletion;
(d) screening in cultured cells or ex vivo the cells of (c) for the phenotypic or genotypic variation relative to the cells of (a);
(e) repeating steps (b)-(d), wherein the location of the second site-specific recombination site is altered relative to that of the first iteration of steps (b)-(d); and
(f) determining the location or identity of the genetic sequence that is correlated with the genotypic or phenotypic variation in the cultured cells or ex vivo, optionally wherein the location of the second site-specific recombination site is altered relative to that of the first iteration of steps (a)-(c) to narrow the size of the deletion that is correlated with the genotypic or phenotypic variation in the cells.

7. A method of identifying a genetic sequence correlated with a recessive genotypic or phenotypic variation, comprising:
screening in cultured cells or ex vivo for genotypic or phenotypic variation between:
(a) cells formerly comprising an introduced recombination site, the region of sequence comprising the introduced recombination site having been deleted, wherein the DNA of the cells further comprises a mutation on a homologous chromosome that was introduced with a second method, wherein the mutation is located at a site on the homologous chromosome corresponding to the deletion; and
(b) cells from the group consisting of:
(i) progenitor cells of the cells of (a) without the non-recombinase-induced deletion in the chromosomal DNA and without the further mutation with the second method;
(ii) progenitor cells of the cells of (a) having the non-recombinase-induced deletion of the recombination site in the chromosomal DNA but absent the further mutation;
(iii) progenitor cells of the cells of (a) having the introduced further mutation but absent the non-recombinase-induced deletion of the recombination site in the chromosomal DNA; and
(iv) cells other than progenitor cells of the cells of (a)
wherein the genetic sequence correlated with the recessive genotypic or phenotypic variation in the cells of (a) is mapped to a region of a genetic sequence correlated with the genotypic or phenotypic variation using the method of claim 6.

8. A population of cells, comprising:
(a) a population of greater than haploid cultured cells with three introduced site-specific recombination sites on a single chromosome, and
(b) a further mutation on a homologous chromosome of the cells at a location bound by sites homologous to two of the introduced recombination sites, optionally wherein the cells have a deletion caused by site-specific recombination between two of the three introduced site-specific recombination sites in the chromosomal DNA.

9. A cell library comprising the cells of claim 8.

10. A method of identifying a recessive genotypic or phenotypic variation, comprising:
screening cells cultured or ex vivo for genotypic or phenotypic variation between:
(a) greater than haploid cells with a first introduced site-specific recombination site in a first homologous chromosome and comprising a deletion in a chromosome region of a second homologous chromosome wherein the deletion in the second homologous chromosome overlaps the first introduced site-specific recombination site in the first homologous chromosome; and
(b) greater than haploid cells of (a) wherein the cells further comprise a deletion in a chromosome region between the first introduced site-specific recombination site and a second introduced site-specific recombination site in the first homologous chromosome,
wherein the correlation of the presence or absence of the genotypic or phenotypic variation in the cells of (b) is correlated with the region of the deletion between the first introduced site-specific recombination site and the second introduced site-specific recombination site.

11. A method of determining the location or identity of a genetic sequence correlated with a recessive genotypic or phenotypic variation in cultured cells or ex vivo comprising:
(a) obtaining greater than haploid cells with a first introduced site-specific recombination site on a first homologous chromosome and comprising a deletion in a chromosome region of a second homologous chromosome, wherein the deletion in the second homologous chromosome overlaps the first introduced site-specific recombination site in the first homologous chromosome, wherein at least a portion of the deleted region is correlated with a genotypic or phenotypic variation;
(b) introducing a second site-specific recombination site onto the first homologous chromosome comprising the first site-specific recombination site in a population of cells of (a);
(c) exposing the cells of (a) to a site-specific recombinase to generate cells comprising at least one deletion on the first homologous chromosome comprising the first and second recombination sites;
(d) screening in cultured cells or ex vivo the cells of (c) for the phenotypic or genotypic variation relative to the cells of (a);
(e) repeating steps (a)-(d), wherein the region of genetic sequence containing the second site-specific recombination site is altered relative to that of the first iteration of steps (a)-(d); and
(f) determining the location or identity of the genetic sequence that is correlated with the genotypic or phenotypic variation in the cultured cells or ex vivo, wherein the presence or absence of the genotypic or phenotypic variation in the cells of (d) indicates the region of the deletion in the homologous chromosome that correlates with the genotypic or phenotypic variation.

## Patentansprüche

1. Verfahren zur Bestimmung des Orts oder der Identität einer genetischen Sequenz, die mit einer rezessiven genotypischen oder phänotypischen Variation korreliert ist, in kultivierten Zellen, wobei die genetische Sequenz sich zwischen zwei inserierten Orts-spezifischen Rekombinations-Stellen befindet, umfassend:
(a) Beschaffung von Zellen, umfassend zwei inserierte Orts-spezifische Rekombinations-Stellen auf einem Chromosom, ferner umfassend eine Mutation, welche mit einer genotypischen oder phänotypischen Variation korreliert ist, wobei sich die Mutation auf einem homologen Chromosom befindet und durch den Ort der Orts-spezifischen Rekombinations-Stellen auf dem Homolog begrenzt wird;
(b) Inserieren einer dritten Orts-spezifischen Rekombinations-Stelle an einem bekannten Ort zwischen den beiden Orts-spezifischen Rekombinations-Stellen in einer Population der Zellen gemäß (a);
(c) Aussetzen der Zellen gemäß (a) einer Orts-spezifischen Rekombinase, um Zellen zu erzeugen, welche mindestens eine Deletion umfassen;
(d) Aussetzen der Zellen gemäß (b) einer Orts-spezifischen Rekombinase, um Zellen zu erzeugen, welche mindestens eine Deletion umfassen;
(e) Screenen, in kultivierten Zellen oder ex vivo, der Deletions-enthaltenden Zellen gemäß (c) relativ zu den Deletions-enthaltenden Zellen gemäß (d) auf das Vorliegen oder die Abwesenheit der phänotypischen oder genotypischen Variation;
(f) Wiederholen der Schritte (a)-(c), wobei die dritte Orts-spezifische Rekombinations-Stelle relativ zu derjenigen der ersten Wiederholung der Schritte (a)-(c) verändert ist;
(g) Bestimmung des Orts oder der Identität einer genetischen Sequenz, welche mit der rezessiven genotypischen oder phänotypischen Variation korreliert ist, in den kultivierten Zellen oder ex vivo.

2. Verfahren gemäß Anspruch 1, worin die dritte Orts-spezifische Rekombinations-Stelle relativ zu derjenigen der ersten Wiederholung der Schritte (a)-(c) verändert ist, um die Größe der Deletion einzuengen, welche mit der genotypischen oder phänotypischen Variation in den Zellen korreliert ist, wobei sich die Mutation an einer Stelle auf dem homologen Chromosom befindet, die der Deletion entspricht.

3. Verfahren zur Identifizierung einer genotypischen oder phänotypischen Variation, umfassend:
Screenen von Zellen, kultiviert oder ex vivo, auf genotypische oder phänotypische Variation zwischen:
(a) mehr als haploiden Zellen mit einer Rekombinase-induzierten Deletion zwischen zwei inserierten Orts-spezifischen Rekombinations-Stellen in der chromosomalen DNA von kultivierten Zellen, wobei die DNA der Zellen ferner eine Mutation in einem Chromosom umfasst, welche zu mindestens einem Teil der Rekombinase-induzierten deletierten Sequenz homolog ist, wobei die weitere Mutation mit Hilfe eines Verfahrens außer Rekombinase-induzierter Deletion eingeführt wurde; und
(b) Zellen ausgewählt aus der Gruppe bestehend aus:
(i) Vorläuferzellen der Zellen gemäß (a) ohne die Rekombinase-induzierte Deletion in der chromosomalen DNA oder die weitere Mutation;
(ii) Vorläuferzellen der Zellen gemäß (a) mit der Rekombinase-induzierten Deletion in der chromosomalen DNA, aber ohne die weitere Mutation;
(iii) Vorläuferzellen der Zellen gemäß (a) mit der eingeführten weiteren Mutation, aber ohne die Rekombinase-induzierte Deletion in der chromosomalen DNA, und
(iv) Zellen außer Vorläuferzellen der Zellen gemäß (a), wobei der Ort oder die Identität der genotypischen oder phänotypischen Variation sich in einer Region einer genetischen Sequenz befindet, welche mit Hilfe des Verfahrens gemäß Anspruch 2 mit der genotypischen oder phänotypischen Variation korreliert wurde.

4. Verfahren gemäß Anspruch 3, worin die weitere Mutation durch chemische Mutagenese erzeugt wurde, oder worin die weitere Mutagenese durch eines oder mehrere von elektromagnetischer Strahlung, Behandlung mit einem Inhibitor der DNA-Reparatur oder Insertionsmutagenese erfolgt ist.

5. Verfahren gemäß Anspruch 4, wobei die weitere Methode der Mutation der kultivierten Zellen gemäß (a) entweder vor, gleichzeitig mit, oder nachfolgend an die Rekombinase-induzierte Deletion durchgeführt wird; oder
wobei die Orts-spezifische Rekombinase Cre-Rekombinase ist; oder
wobei die Zellen vor der Mutagenese eng einen diploiden Karyotyp einhalten; oder
worin die phänotypische Variation ein Mitglied ist, ausgewählt aus der Gruppe bestehend aus einer veränderten Fähigkeit, Kolonien in einem halbfesten Medium zu bilden, veränderter Resistenz gegen Chemotherapeutika, veränderter Induktion von Neo-Angiogenese und einer veränderten Fähigkeit zu metastasieren.

6. Verfahren zur Bestimmung des Orts oder der Identität einer genetischen Sequenz, welche mit einer rezessiven genotypischen oder phänotypischen Variation korreliert ist, in kultivierten Zellen oder ex vivo, umfassend:
(a) Beschaffen von Zellen, welche eine inserierte erste Orts-spezifische Rekombinations-Stelle umfassen, ferner umfassend eine Mutation, die mit einer genotypischen oder phänotypischen Variation korreliert ist, wobei die Mutation sich auf einem homologen Chromosom befindet;
(b) Einführen einer zweiten Orts-spezifischen Rekombinations-Stelle in das Chromosom, umfassend die erste Orts-spezifische Rekombinations-Stelle in einer Population von Zellen gemäß (a), wobei der Ort der Mutation auf dem homologen Chromosom durch den Ort der Orts-spezifischen Rekombinations-Stellen auf dem Homolog begrenzt ist;
(c) Aussetzen der Zellen gemäß (b) einer Orts-spezifischen Rekombinase, um Zellen zu erzeugen, welche mindestens eine Deletion umfassen;
(d) Screenen in kultivierten Zellen, oder ex vivo, der Zellen gemäß (c) auf die phänotypische oder genotypische Variation relativ zu den Zellen gemäß (a);
(e) Wiederholen der Schritte (b)-(d), wobei der Ort der zweiten Orts-spezifischen Rekombinations-Stelle relativ zu demjenigen der ersten Wiederholung der Schritte (b)-(d) verändert wird; und
(f) Bestimmen des Orts oder der Identität der genetischen Sequenz, welche mit der genotypischen oder phänotypischen Variation korreliert ist, in den kultivierten Zellen oder ex vivo, wobei gegebenenfalls der Ort der zweiten Orts-spezifischen Rekombinations-Stelle relativ zu demjenigen der ersten Wiederholung der Schritte (a)-(c) verändert wird, um die Größe der Deletion einzuengen, welche mit der genotypischen oder phänotypischen Variation in den Zellen korreliert ist.

7. Verfahren zur Identifizierung einer genetischen Sequenz, die mit einer rezessiven genotypischen oder phänotypischen Variation korreliert ist, umfassend:
Screenen, in kultivierten Zellen oder ex vivo, auf genotypischer oder phänotypischer Variation zwischen:
(a) Zellen, welche ehemals eine eingeführte Rekombinations-Stelle umfassten, wobei die Region der Sequenz, welche die eingeführte Rekombinations-Stelle umfasste, deletiert worden ist, wobei die DNA der Zellen ferner eine Mutation auf einem homologen Chromosom umfasst, die mit Hilfe eines zweiten Verfahrens eingeführt wurde, wobei die Mutation sich an einer Stelle auf dem homologen Chromosom befindet, welche der Deletion entspricht; und
(b) Zellen aus der Gruppe bestehend aus:
(i) Vorläuferzellen der Zellen gemäß (a) ohne die nicht-Rekombinase-induzierte Deletion in der chromosomalen DNA und ohne die weitere Mutation mit Hilfe des zweiten Verfahrens;
(ii) Vorläuferzellen der Zellen gemäß (a) mit der nicht-Rekombinase-induzierten Deletion der Rekombinations-Stelle in der chromosomalen DNA, aber ohne die weitere Mutation;
(iii) Vorläuferzellen der Zellen gemäß (a) mit der eingeführten weiteren Mutation, aber ohne die nicht-Rekombinase-induzierte Deletion der Rekombinations-Stelle in der chromosomalen DNA; und
(iv) Zellen außer den Vorläuferzellen der Zellen gemäß (a), wobei die genetische Sequenz, welche mit der rezessiven genotypischen oder phänotypischen Variation in den Zellen gemäß (a) korreliert ist, auf eine Region einer genetischen Sequenz kartiert wird, welche mit Hilfe des Verfahrens gemäß Anspruch 6 mit der genotypischen oder phänotypischen Variation korreliert wurde.

8. Population von Zellen, umfassend:
(a) eine Population von mehr als haploiden kultivierten Zellen mit drei eingeführten Orts-spezifischen Rekombinations-Stellen auf einem einzigen Chromosom, und
(b) einer weiteren Mutation auf einem homologen Chromosom der Zellen an einem Ort, welcher durch Stellen begrenzt wird, welche homolog zu zwei der eingeführten Rekombinations-Stellen sind, wobei die Zellen gegebenenfalls eine Deletion besitzen, welche durch Orts-spezifische Rekombination zwischen zwei der drei eingeführten Orts-spezifischen Rekombinations-Stellen in der chromosomalen DNA entstanden ist.

9. Zellbibliothek umfassend die Zellen gemäß Anspruch 8.

10. Verfahren zur Identifizierung einer rezessiven genotypischen oder phänotypischen Variation, umfassend:
Screenen von Zellen, kultiviert oder ex vivo, auf genotypische oder phänotypische Variation zwischen:
(a) mehr als haploiden Zellen mit einer ersten eingeführten Orts-spezifischen Rekombinations-Stelle in einem ersten homologen Chromosom und umfassend eine Deletion in einer Chromosomenregion eines zweiten homologen Chromosoms, wobei die Deletion im zweiten homologen Chromosom mit der ersten eingeführten Orts-spezifischen Rekombinations-Stelle im ersten homologen Chromosom überlappt; und
(b) mehr als haploiden Zellen gemäß (a), wobei die Zellen ferner eine Deletion in einer Chromosomenregion zwischen der ersten eingeführten Orts-spezifischen Rekombinations-Stelle und einer zweiten eingeführten Orts-spezifischen Rekombinations-Stelle in dem ersten homologen Chromosom umfassen,
wobei die Korrelation des Vorliegens oder der Abwesenheit der genotypischen oder phänotypischen Variation in den Zellen gemäß (b) mit der Region der Deletion zwischen der ersten eingeführten Orts-spezifischen Rekombinations-Stelle und der zweiten eingeführten Orts-spezifischen Rekombinations-Stelle korreliert wird.

11. Verfahren zur Bestimmung des Orts oder der Identität einer genetischen Sequenz, welche mit einer rezessiven genotypischen oder phänotypischen Variation korreliert ist, in kultivierten Zellen oder ex vivo, umfassend:
(a) Beschaffung mehr als haploider Zellen mit einer ersten eingeführten Orts-spezifischen Rekombinations-Stelle auf einem ersten homologen Chromosom und umfassend eine Deletion in einer Chromosomenregion eines zweiten homologen Chromosoms, wobei die Deletion im zweiten homologen Chromosom mit der ersten eingeführten Orts-spezifischen Rekombinations-Stelle im ersten homologen Chromosom überlappt, wobei mindestens ein Teil der deletierten Region mit einer genotypischen oder phänotypischen Variation korreliert ist;
(b) Einführen einer zweiten Orts-spezifischen Rekombinations-Stelle in das erste homologe Chromosom, umfassend die erste Orts-spezifische Rekombinations-Stelle in einer Population von Zellen gemäß (a);
(c) Aussetzen der Zellen gemäß (a) einer Orts-spezifischen Rekombinase, um Zellen zu erzeugen, welche mindestens eine Deletion auf dem ersten homologen Chromosom umfassen, umfassend die erste und zweite Rekombinations-Stelle;
(d) Screenen der Zellen gemäß (c) auf die phänotypische oder genotypische Variation relativ zu den Zellen gemäß (a) in kultivierten Zellen oder ex vivo;
(e) Wiederholung der Schritte (a)-(d), wobei die Region der genetischen Sequenz, welche die zweite Orts-spezifische Rekombinations-Stelle enthält, relativ zu derjenigen der ersten Wiederholung der Schritte (a)-(d) verändert wird; und
(f) Bestimmen des Orts oder der Identität der genetischen Sequenz, die mit der genotypischen oder phänotypischen Variation korreliert ist, in den kultivierten Zellen oder ex vivo, wobei die Gegenwart oder Abwesenheit der genotypischen oder phänotypischen Variation in den Zellen gemäß (d) die Region der Deletion im homologen Chromosom aufzeigt, die mit der genotypischen oder phänotypischen Variation korreliert.

## Revendications

1. Procédé pour déterminer l'emplacement ou l'identité d'une séquence génétique corrélée avec une variation génotypique ou phénotypique récessive chez des cellules cultivées, ladite séquence génétique étant située entre deux sites de recombinaison à spécificité de site insérés, lequel procédé comporte les étapes suivantes :
a) obtenir des cellules au sein desquelles deux sites de recombinaison à spécificité de site ont été insérés sur un chromosome et qui présentent en outre une mutation corrélée avec une variation génotypique ou phénotypique, laquelle mutation se trouve sur un chromosome homologue et attachée près de l'emplacement des sites de recombinaison à spécificité de site sur l'homologue ;
b) insérer, chez une population de cellules de l'étape (a), un troisième site de recombinaison à spécificité de site en un emplacement connu situé entre les deux sites de recombinaison à spécificité de site ;
c) exposer les cellules de l'étape (a) à une recombinase à spécificité de site, afin de générer des cellules portant au moins une délétion ;
d) exposer les cellules de l'étape (b) à une recombinase à spécificité de site, afin de générer des cellules portant au moins une délétion ;
e) rechercher par criblage sur cellules cultivées ou ex vivo, dans les cellules porteuses de délétion de l'étape (c) par rapport aux cellules porteuses de délétion de l'étape (d), la présence ou l'absence de la variation phénotypique ou génotypique ;
f) répéter les étapes (a)-(c), en modifiant le troisième site de recombinaison à spécificité de site par rapport à celui de la première itération des étapes (a)-(c) ;
g) déterminer l'emplacement ou l'identité d'une séquence génétique qui est corrélée avec la variation génotypique ou phénotypique récessive, chez les cellules cultivées ou ex vivo.

2. Procédé conforme à la revendication 1, dans lequel on modifie le troisième site de recombinaison à spécificité de site, par rapport à celui de la première itération des étapes (a)-(c), de manière à faire diminuer la taille de la délétion qui est corrélée avec la variation génotypique ou phénotypique chez les cellules, étant entendu que la mutation se trouve, sur le chromosome homologue, en un site correspondant à la délétion.

3. Procédé d'identification d'une variation génotypique ou phénotypique, comportant le fait de rechercher, par criblage sur cellules cultivées ou ex vivo, une variation génotypique ou phénotypique entre :
a) des cellules plus qu'haploïdes présentant une délétion induite par recombinase, située entre deux sites de recombinaison à spécificité de site insérés dans l'ADN chromosomique de cellules cultivées, étant entendu que l'ADN des cellules comporte une mutation supplémentaire dans un chromosome homologue comportant au moins une partie de la séquence éliminée par délétion induite par recombinase, laquelle mutation supplémentaire a été introduite autrement que par délétion induite par recombinase ;
b) et des cellules choisies dans l'ensemble formé par les suivantes :
i) des cellules progénitrices des cellules (a), sans la délétion induite par recombinase dans l'ADN chromosomique ni la mutation supplémentaire,
ii) des cellules progénitrices des cellules (a), présentant la délétion induite par recombinase dans l'ADN chromosomique, mais sans la mutation supplémentaire,
iii) des cellules progénitrices des cellules (a), présentant la mutation supplémentaire, mais sans la délétion induite par recombinase dans l'ADN chromosomique,
iv) et des cellules autres que des cellules progénitrices des cellules (a) ;
étant entendu que l'emplacement ou l'identité de la variation génotypique ou phénotypique est localisé(e) en une région d'une séquence génétique corrélée avec la variation génotypique ou phénotypique selon un procédé conforme à la revendication 2.

4. Procédé conforme à la revendication 3, pour lequel la mutation supplémentaire a été produite par mutagenèse chimique, ou pour lequel la mutation supplémentaire est l'effet de l'un ou plusieurs des procédés de mutagenèse suivants : exposition à un rayonnement électromagnétique, traitement avec un agent inhibant la réparation de l'ADN, et mutagenèse par insertion.

5. Procédé conforme à la revendication 4, dans lequel
- le procédé produisant la mutation supplémentaire chez les cellules (a) cultivées est mis en oeuvre antérieurement, concomitamment ou postérieurement à la délétion induite par recombinase ;
- ou la recombinase à spécificité de site est la recombinase Cre ;
- ou les cellules d'avant la mutagenèse sont très près de présenter un cariotype diploïde ;
- ou la variation phénotypique est un élément de l'ensemble formé par une altération de la capacité à proliférer en colonies sur milieu semi-solide, une altération de la résistance à des agents chimiothérapeutiques, une altération du pouvoir d'induire une néo-angiogenèse, et une altération de la capacité à produire des métastases.

6. Procédé pour déterminer l'emplacement ou l'identité d'une séquence génétique corrélée avec une variation génotypique ou phénotypique récessive, chez des cellules cultivées ou ex vivo, lequel procédé comporte les étapes suivantes :
a) obtenir des cellules au sein desquelles a été inséré un premier site de recombinaison à spécificité de site et qui présentent en outre une mutation qui est corrélée avec une variation génotypique ou phénotypique, laquelle mutation se trouve sur un chromosome homologue ;
b) introduire chez une population de cellules de l'étape (a), sur le chromosome portant le premier site de recombinaison à spécificité de site, un deuxième site de recombinaison à spécificité de site, étant entendu que l'emplacement de la mutation sur le chromosome homologue est attaché près de l'emplacement des sites de recombinaison à spécificité de site sur l'homologue ;
c) exposer les cellules de l'étape (b) à une recombinase à spécificité de site, afin de générer des cellules portant au moins une délétion ;
d) rechercher par criblage sur cellules cultivées ou ex vivo, dans les cellules de l'étape (c), la variation phénotypique ou génotypique par rapport aux cellules de l'étape (a) ;
e) répéter les étapes (b)-(d), en modifiant l'emplacement du deuxième site de recombinaison à spécificité de site par rapport à celui de la première itération des étapes (b)-(d) ;
f) et déterminer l'emplacement ou l'identité de la séquence génétique qui est corrélée avec la variation génotypique ou phénotypique, chez les cellules cultivées ou ex vivo ;
étant entendu que, en option, on modifie l'emplacement du deuxième site de recombinaison à spécificité de site, par rapport à celui de la première itération des étapes (a)-(c), de manière à faire diminuer la taille de la délétion qui est corrélée avec la variation génotypique ou phénotypique chez les cellules.

7. Procédé d'identification d'une séquence génétique corrélée avec une variation génotypique ou phénotypique récessive, comportant le fait de rechercher, par criblage sur cellules cultivées ou ex vivo, une variation génotypique ou phénotypique entre :
a) des cellules chez lesquelles avait été antérieurement introduit un site de recombinaison et d'où a été éliminée par délétion la région de séquence comportant ce site de recombinaison introduit, étant entendu que l'ADN des cellules comporte en outre, sur un chromosome homologue, une mutation qui a été introduite par un deuxième procédé, laquelle mutation se trouve, sur le chromosome homologue, en un site correspondant à la délétion ;
b) et des cellules choisies dans l'ensemble formé par les suivantes :
i) des cellules progénitrices des cellules (a), sans la délétion non induite par recombinase dans l'ADN chromosomique et sans la mutation supplémentaire introduite par le deuxième procédé,
ii) des cellules progénitrices des cellules (a), présentant la délétion, non induite par recombinase, du site de recombinaison dans l'ADN chromosomique, mais sans la mutation supplémentaire,
iii) des cellules progénitrices des cellules (a), présentant la mutation supplémentaire introduite, mais sans la délétion, non-induite par recombinase, du site de recombinaison dans l'ADN chromosomique,
iv) et des cellules autres que des cellules progénitrices des cellules (a) ;
étant entendu que la séquence génétique corrélée avec la variation génotypique ou phénotypique récessive dans les cellules (a) est mise en correspondance avec une région d'une séquence génétique corrélée avec la variation génotypique ou phénotypique selon un procédé conforme à la revendication 6.

8. Population de cellules comprenant
a) une population de cellules cultivées, plus qu'haploïdes, porteuses de trois sites de recombinaison à spécificité de site introduits sur un seul chromosome,
b) et une mutation supplémentaire sur un chromosome homologue des cellules, en un emplacement attaché près des sites homologues à deux des sites de recombinaison introduits,
étant entendu que, en option, les cellules présentent une délétion provoquée par recombinaison à spécificité de site entre deux des trois sites de recombinaison à spécificité de site introduits dans l'ADN chromosomique.

9. Bibliothèque de cellules comprenant des cellules conformes à la revendication 8.

10. Procédé d'identification d'une variation génotypique ou phénotypique récessive, comportant le fait de rechercher, par criblage sur cellules cultivées ou ex vivo, une variation génotypique ou phénotypique entre :
a) des cellules plus qu'haploïdes présentant un premier site de recombinaison à spécificité de site introduit dans un premier chromosome homologue et présentant une délétion dans une région chromosomique d'un deuxième chromosome homologue, étant entendu que cette délétion dans le deuxième chromosome homologue chevauche le premier site de recombinaison à spécificité de site introduit dans le premier chromosome homologue ;
b) et des cellules plus qu'haploïdes (a), qui présentent en outre une délétion dans une région chromosomique entre le premier site de recombinaison à spécificité de site introduit et un deuxième site de recombinaison à spécificité de site introduit dans le premier chromosome homologue,
étant entendu que la présence ou l'absence de la variation génotypique ou phénotypique dans les cellules (b) est corrélée avec la région de la délétion entre le premier site de recombinaison à spécificité de site introduit et le deuxième site de recombinaison à spécificité de site introduit.

11. Procédé pour déterminer l'emplacement ou l'identité d'une séquence génétique corrélée avec une variation génotypique ou phénotypique récessive chez des cellules cultivées ou ex vivo, lequel procédé comporte les étapes suivantes :
a) obtenir des cellules plus qu'haploïdes présentant un premier site de recombinaison à spécificité de site introduit dans un premier chromosome homologue et présentant une délétion dans une région chromosomique d'un deuxième chromosome homologue, étant entendu que cette délétion dans le deuxième chromosome homologue chevauche le premier site de recombinaison à spécificité de site introduit dans le premier chromosome homologue, et qu'au moins une partie de la région éliminée par délétion est corrélée avec une variation génotypique ou phénotypique ;
b) introduire, chez une population de cellules de l'étape (a), un deuxième site de recombinaison à spécificité de site dans le premier chromosome homologue comportant le premier site de recombinaison à spécificité de site ;
c) exposer les cellules de l'étape (a) à une recombinase à spécificité de site, afin de générer des cellules portant au moins une délétion sur le premier chromosome homologue comportant les premier et deuxième sites de recombinaison ;
d) rechercher par criblage sur cellules cultivées ou ex vivo, dans les cellules de l'étape (c), la variation phénotypique ou génotypique, par rapport aux cellules de l'étape (a) ;
e) répéter les étapes (a)-(d), en modifiant la région de séquence génétique qui comporte le deuxième site de recombinaison à spécificité de site, par rapport à celle de la première itération des étapes (a)-(d) ;
f) et déterminer l'emplacement ou l'identité de la séquence génétique qui est corrélée avec la variation génotypique ou phénotypique, chez les cellules cultivées ou ex vivo, étant entendu que la présence ou l'absence de la variation génotypique ou phénotypique chez les cellules de l'étape (d) indique la région de la délétion dans le chromosome homologue qui est corrélée avec la variation génotypique ou phénotypique.
